# EUROPEAN PATENT APPLICATION

(11) **EP 2 289 553 A1**
(43) Date of publication of application: **02.03.2011**
(21) Application number: 09011190.7
(22) Date of filing: 01.09.2009
(51) Int. Cl.: A61K 41/00, A61K 49/00

(54) **Dispersant stabilization of inorganic non-metallic particles**

(71) Applicant: ETH Zurich, 8092 Zurich (CH)
(72) Inventor: Amstad, Esther, 8057 Zürich (CH); Reimhul, Erik, 8050 Zürich (CH); Textor, Marcus, 8200 Schaffhausen/SH (CH)

(57) **Abstract**

1. A dispersant for stabilizing nanoparticles, in particular inorganic non-metallic nanoparticles is described. Said dispersant comprises at least one anchor group A, and at least one spacing group R covalently linked to said anchor group, said anchor group A is an unsaturated at least partially conjugated substituted six-membered homocycle or heterocycle and comprises two surface interacting oxygen or hydroxy substituents on adjacent carbon atoms in the ring. In the case of a homocyclic ring said ring comprises at least one further electronegative substituent on the ring, in the case of a heterocyclic ring the further electronegative substituent on the ring is optional.

## Description

### Technical Field

The present invention regards the stabilization of inorganic non-metallic particles with dispersants, in particular the stabilization of nanoparticles such as superparamagnetic iron oxide nanoparticles (SPIONs) with catechol derived dispersants.

### Background Art

The number of potential applications of superparamagnetic iron oxide nanoparticles (SPIONs) especially, but not exclusively, in the biomedical field is rapidly increasing with emerging technologies to tune and control their bulk and, even more importantly, surface properties. Due to the high biocompatibility of iron oxide nanoparticles and their superparamagnetic behavior, they are often used as, e.g., magnetic resonance (MR) contrast agents to achieve cellular or even molecular resolution and for cell tracking and separation purposes, and are emerging in hybrid and smart material applications dispersed in polymer matrices. However, the quality and performance of such iron oxide nanoparticles crucially depend on nanoparticle size, stability, dispersant layer thickness and control over their interfacial chemistry.

Iron oxide nanoparticles are typically sterically stabilized with dispersants if used for biomedical applications. Dispersants, such as dextran, which lack a well defined high affinity anchor group lead to broad particle size distributions with average hydrodynamic diameters many times larger than the iron oxide core diameter. Needless to say the resulting interfacial chemistry is poorly defined and thus prevents efficient incorporation and controlled presentation of ligands. Steric stability can also be provided by low molecular weight dispersants strongly bound to the iron oxide nanoparticle surface at high packing densities. Low molecular weight dispersants which consist of one high affinity anchor group covalently linked to a hydrophilic polymer can be grafted to iron oxide nanoparticle surfaces in a well defined way without the need for further *in situ* chemistry [1-5]. Dopamine, a derivative of the amino acid DOPA which is abundantly present in the mussel adhesive protein Mytilus edulis, is the most considered high affinity binding group to stabilize iron oxide nanoparticles in water [4] and physiologic buffers [2,3]. Nevertheless, the suitability of dopamine as a high affinity anchor for iron oxide nanoparticle stabilization is debated mainly because dopamine degradation has been claimed upon adsorption on iron oxide nanoparticles [6]. Furthermore, aliquotted dopamine has been shown to be prone to oxidation. Despite the versatility of this method to disperse SPIONs and the tough requirements regarding affinity and stability of the anchor group, there is still a need for anchor groups that provide improved nanoparticle stability, in particular particle stability at 37 °C or higher that is important for *in vivo* applications such as imaging and stimulated drug release.

### Disclosure of the Invention

Hence, it is a general object of the invention to provide a dispersant that is irreversibly binding to inorganic non-metallic particles, preferably nanoparticles, in particular to iron oxide nanoparticles.

It is a further object of the present invention to provide methods for preparing stabilized and optionally functionalized inorganic non-metallic particles.

It is a further object of the present invention to provide stabilized particle dispersions.

It is yet another object of the present invention to provide several uses for the stabilized particles.

Now, in order to implement these and still further objects of the invention, which will become more readily apparent as the description proceeds, the dispersant of the present invention is manifested by the features that it comprises at least one anchor group A, and at least one linear or branched spacing group R covalently linked to each other directly or via a connecting group,
said anchor group A being an unsaturated at least partially conjugated substituted six-membered homocycle or heterocycle,
said anchor group A comprising two surface interacting oxygen or hydroxy substituents on adjacent carbon atoms in the ring,
said homocyclic ring comprising at least one further electronegative substituent on the ring, in the case of a further hydroxy substituent in meta position to the spacing group at least two electronegative substituents
said heterocyclic ring comprising at least one nitrogen and optionally at least one electronegative substituent on the ring.

In one embodiment, said anchor group comprises a covalently linked connecting group -C(L4)-C(L5)- for connecting the spacing group R to the anchor group, and
said spacing group R is covalently linked to said anchor group via said connecting group. L4 and L5 are individually selected from hydrogen and electronegative groups, preferably -COOH.

Unless otherwise defined, *electronegative substituent* means that the substituent has higher electronegativity than the atom to which it is bound

Preferred electronegative groups are selected from the group consisting of F, Cl, Br, I, NO₂, COOH, OH, SO₄, PO₄.

The spacing group R may further be defined as a group Sₙ-Fₘ, wherein S is a linear or branched spacer, F is a functional group that is independently from each other selected from the group consisting of functionalities E, terminating groups T such as methyl groups and hydrogen, anchor groups A and combinations thereof, n and m are integers, n is selected from 0 or 1, m corresponds to the number of branches of S + 1 and is at least 1.

The term branched as it is used in the scope of the present invention encompasses the terms often used in connection with non-linear polymers such as dendritic, hyperbranched etc.

Dispersants, spacing groups or spacer branches carrying a functionalization E are also termed functionalized, while dispersants, spacing groups and spacer branches carrying a terminating group are termed unfunctionalized.

Such dispersant is preferably suitable for the stabilization of inorganic non-metallic, especially ceramic, particles.

Preferred are low molecular weight dispersants having a molecular weight that irrespective of the number of anchors bound to the spacer, result in a spacer layer able to extend into the solvent far enough to overcome the attractive forces between the particles such as Van der Waals forces, thus providing steric stability to so-stabilized particle dispersions. For dispersants consisting of one anchor group and one spacer group, molecular weights down to about 800 Da for hydrophilic sterically repulsive spacers such as poly(ethylene glycol) (PEG) already provide good results. Preferred, however, are larger spacers resulting in dispersants having molecular weights in the low kDa range, e.g. in the range of 1.5 to 8 kDa, more preferred in the range of 1.5 to 5 kDa. These ranges are especially suitable for medical applications. For other applications higher kDa ranges may be preferred. If several anchors are present, the spacers connecting these anchor groups may be small, whereas the "free" spacing group or free branch of the spacer, i.e. a spacer connected to a functionality E or a terminating group T, should be long or bulky enough to provide for the needed inter-particle distance.

In another preferred embodiment, the six-membered homocycle or heterocycle is selected from quinolines, pyridines and/or derivatives thereof such as mimosine, and/or hydroxypyridines,

The high affinity anchor groups of the catechol type are conjugated organic molecules substituted such that improved electron transfer between the adsorbed anchor groups and the surface of inorganic non-metallic particles occurs. Such anchor groups (also referred to as biomimetic anchor groups) may be catechol derivatives, i.e. an aromatic ring as shown below wherein all Zs are carbons.

If all Zs are carbons, at least one of the substituents L₁-L₃ has to be an electronegative group such as but not limited to F, Cl, Br, I, NO₂, COOH, OH, SO₄, PO₄. In addition to the aforementioned list of electronegative substituents the other substituents are hydrogens. In case that L₂= OH, L₁ and/or L₃ must also be an electronegative group. L₄ and L₅ may independently from each other be selected from hydrogen and electrophilic substituents such as COOH.

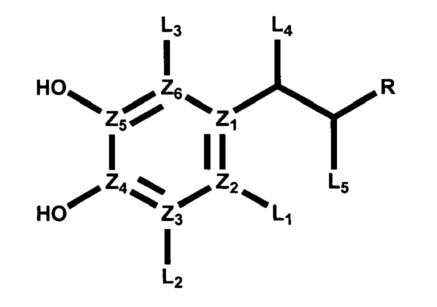

The catechol type anchor groups in particular also encompass 1,2-quinones, 1,2-semiquinones and combinations thereof. L₁ to L₅ are as defined above. The electron displacement in semiquinones and quinones is further illustrated below.

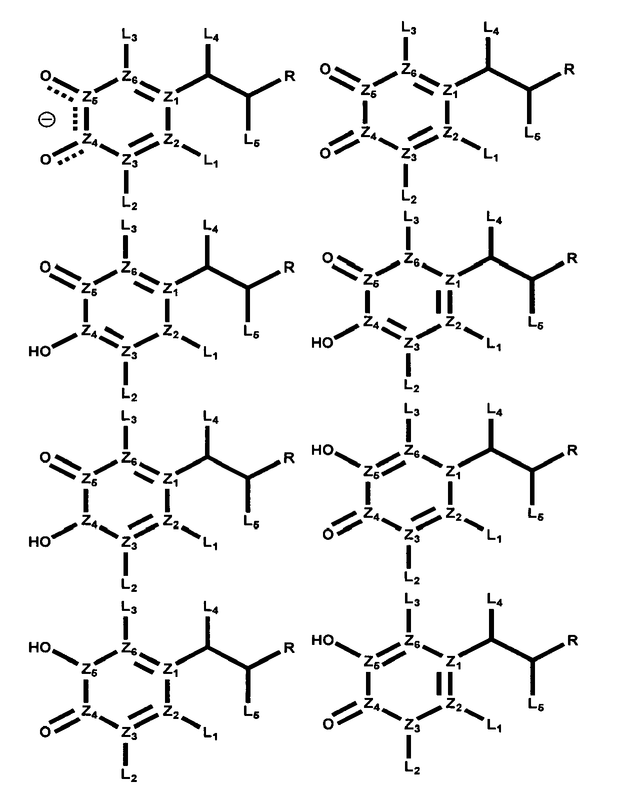

In case of biomimetic anchor groups with a six-membered at least partially conjugated heterocycle, preferred heterocycles are selected from pyridines, and/or derivatives thereof such as mimosine and/or hydroxypyridines. Other heterocycles are those with one of the above shown formulas of catechol, quinone and semiquinone type anchor groups wherein each of Z₁, Z₂, Z₃ and Z₆ is selected from the group consisting of C, N with the proviso that at most two Z are N and Z₄ and Z₅ are C,. L₁, L₂ and L₃ can be protons and/or electrophilic substituents including but not limited to F, Cl, Br, I, NO₂, COOH, OH, SO₄, PO₄. L₄ and L₅ can be either protons and/or electrophilic substituents e.g. COOH.

In a spacing group -Sₙ-Fₘ as defined above the spacer S can be linear (no branch) with m = 1, or branched. In the case of a branched spacer, m corresponds to the number of branches of the spacer plus 1. For a once branched S, two functional groups F are present and the following combinations are possible: A,A; A,E; A,T; E,T; E,E; and T,T. Branched spacers can also be anchored to one or more inorganic non-metallic particles through multiple of said anchor groups. These multiple anchor groups bound to the same spacer can be either identical or a combination of different anchors. Different anchors could e.g. consist of a combination of one or multiple homocycles and one or multiple heterocycles, different homocycles or different heterocycles. The anchors can be either directly linked to each other, linked through a small spacer which provides conformational freedom to the anchors or through one or more large spacers.

The substituent R may be a group Sₙ-Fₘ with a linear or branched spacer (S) which is covalently linked to the anchor group via the connecting group (-C(L4)-C(L5)-). The connection of the spacer to the connecting group may be e.g. through amide, ester, urethane or azide bindings. Some not limiting examples for spacers are hydrophilic, biocompatible polymers and/or oligomers including but not limited to poly(ethylene glycol) (PEG), linear or branched, e.g. dendritic polymer chains.

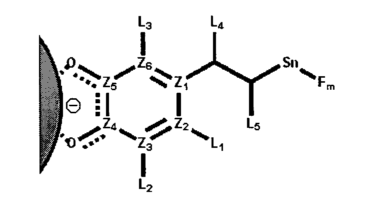

Other examples for spacers (S) are hydrophobic linear or branched spacers including but not limited to alkyl chains, or responsive polymers e.g. thermo-, light- or pH-responsive polymers including but not limited to e.g. poly(N-isopropylacrylamide), and poly(methyl oxazolines) that can be used to prepare stimuli responsive materials (e.g in case of thermo- and pH-responsive polymers, or for producing a cross-linked film (light or thermo induced cross-linking).

The dispersants of the present invention can be functionalized. Different functionalities (E) can be linked to the spacer or alternatively directly to the connecting group. Besides of providing stability, it is possible to irreversibly bind functional groups to the particles at the desired surface concentration through the inventive anchor group.

Functional groups or functionalities (E) can be
ligands for targeting purposes such as biotin, antibodies, proteins, DNA, RNA, aptamers and/or Fab fragments, or
ligands for additional imaging modalities such as, but not limited to, fluorophores, quantum dots, radioactive tracers, chelating agents, MR active ions, Raman and/or IR active groups, or
ligands for further binding and crosslinking including but not limited to acrylates and ion complexers like terpyridin.

Such dispersants can be used to produce inorganic non-metallic particles such as oxides, carbides and nitrides that are individually stabilized after completion of the particle synthesis.

Such dispersants are preferably used to stabilize particles that are difficult to stabilize such as small particles, in particular nanoparticles, such as nanoparticles selected from oxides, oxides/hydroxides, carbides and nitrides.

Small particles as used herein usually have diameters up to 1 µm, preferably up to 500 nm and the term nanoparticle as used herein refers to particles with diameters up to 100 nm, more preferred with diameters up to 50 nm, much preferred up to 10 nm. Wherever used in the scope of the present invention, the term particle encompasses small particles and nanoparticles and in a preferred embodiment is limited to small particles and nanoparticles. Much preferred particles in the scope of this invention are nanoparticles. All particle sizes mentioned above refer to the core particles, i.e the particles without dispersant shell.

One method suitable for producing stabilized particles is by a simple grafting to approach where no special equipment is needed. Particle stabilization can be performed at low temperatures without the risk of temperature induced ligand degradation.

Inorganic non-metallic particles can also be individually stabilized using grafting from techniques such as free radical polymerization from initiators irreversibly immobilized on the particle surface using the inventive anchor groups.

By co-adsorption of different dispersants and/or dispersants which have the same anchor group but different functionalities, particles with multiple functionalities can be generated and the surface density can be tuned by molar ratios of differently functionalized and/or unfunctionalized dispersants and through successive addition of differently functionalized and unfunctionalized dispersants to non-metallic inorganic particles during the stabilization process.

If the functionality is a reactive group, particles can be further functionalized by linking the desired functionality to the reactive end of dispersants of already stabilized particles.

By using the dispersants of the present invention, particle size, stability, zeta potential and interparticle interactions can be tailored by individually controlling the core size and choosing the appropriate molecular weight and molecular configuration (e.g. linear, branched or dendritic) of the spacers in the dispersant. Furthermore, particle size and stability can also be tailored by adjusting the salt concentration and solvent quality with respect to the spacer molecule.

It is also possible to prepare particles with dynamic shell structures that are able to respond to external changes such as temperature, pH, salt concentration or to illumination with light. Such particles may be produced by co-adsorption using dispersants with responsive spacing groups differing in their molecular weights, chemical composition and properties or by adsorption of amphiphilic block copolymers. By both methods e.g. amphiphilic particles that are soluble in polar and non-polar liquids may be generated.

Using dispersants of the present invention it is also possible to reduce non-specific adsorption of molecules, in particular biomolecules such as, but not limited to, proteins, peptides, opsonins, and thus to increase e.g. bood half life time of stabilized particles. This may be achieved by stabilizing particles with stealth dispersants, e.g., but not limited to, poly(ethylene glycol) and poly(methyloxazoline) covalently linked to anchor groups as defined herein. This effect is due to the high dispersant packing densities that can be achieved with such dispersants having linear or branched, also comprising dendritic or hyperbranched, spacing groups or a combination of such spacing groups.

The protein resistance of inorganic non-metallic particles stabilized and functionalized with dispersants of the present invention can be retained even after functionalization with other labels such as but not limited to fluorophores, quantum dots, radioactive tracers and/or MR active ions. Such particles can e.g. be produced by co-adsorption of functionalized and unfunctionalized dispersants, e.g. by using functionalized and unfunctionalized dispersants having different lengths of the spacer groups and wherein the spacer of the functionalized dispersant is short compared to that of non-functional dispersants. Alternatively these additional labels can be covalently linked directly to the connecting group described herein.

Magnetic resonance contrast agents having inorganic non-metallic cores including, but not limited to FeO_{X}, FeOOH and FeC_{X}, can be stabilized with the dispersants of the present invention. Due to the high affinity of the anchor groups described herein, such particles are stable at extreme dilution (i.e. the dispersant does not desorb) and upon heating above body temperature. Preferred anchor groups for such application are nitrocatechols, nitrosemiquinones and/or nitroquinones. The stabilization of such core particles may be obtained by co-adsorption of functional stealth dispersants, including, but not limited to spacing groups comprising or being end-functionalized poly(ethylene glycol).

Also particles for cell and molecular separation having inorganic non-metallic cores including, but not limited to, FeO_{X}, FeOOH and FeC_{X}, can be stabilized with the dispersants of the present invention. Due to the high affinity of the anchor groups described herein, such particles are stable at extreme dilution (i.e. the dispersant dies not desorb) and upon heating above body temperature. Preferred anchor groups for such application are nitrocatechols, nitrosemiquinones and/or nitroquinones. The stabilization of such core particles may be obtained by co-adsorption of functional e.g. thermo-, pH-, light-responsive polymers or end-functionalized stabilizing polymers.

Another aspect of the present invention are biocompatible inorganic non-metallic particles stabilized and functionalized with dispersants of the present invention that can be specifically targeted *in vitro* and *in vivo* by covalently linking ligands as functionalizing group E such as but not limited to peptides, antibodies, aptamers, DNA, RNA and/or ion-complexing groups such as NTA, diethylenetriamino-pentaacetic acid (DTPA) and terpyridines.

Another application of the inventive dispersants is in tailoring the blood half-life time of inorganic non-metallic particles. This can be done by adjusting the spacer molecule of the inventive dispersant used to stabilize the particles, namely the molecular weight and the functional groups of the spacer molecule.

Inorganic non-metallic magnetic particles stabilized and, if needed functionalized, with dispersants of the present invention can be used in hyperthermia e.g. for but not limited to cancer treatment using alternating magnetic fields.

Inorganic non-metallic magnetic particles stabilized and, if needed functionalized, with dispersants of the present invention can be used to generate heat by applying an alternating magnetic field and thus can be used for the creation of smart and/or reversibly responsive materials. The reversibility of the responses is ensured by the high affinity of the anchor groups even at elevated temperatures which prevent dispersant desorption.

Inorganic non-metallic particles stabilized and, if needed functionalized, with dispersants of the present invention can also be used to generate heat by application of high intensity electromagnetic fields, e.g. by laser irradiation, and thus can be used for the creation of smart and/or reversibly responsive materials. Also in this application the reversibility of the responses is ensured by the high affinity of the anchor groups described above even at elevated temperatures which prevent dispersant desorption.

Charged or magnetic inorganic non-metallic particles stabilized and, if needed functionalized, with dispersants of the present invention can when dispersed in a material, e.g. a polymer or hydrogel, also be used to apply force by application of external electromagnetic fields. The high affinity of the anchor groups described above will prevent a properly stabilized particle from precipitating out of the material at such applied force.

Another field of application is in the creation of nano, meso and microporous materials by any deposition method using solvents requiring stably dispersed particles. In this application, inorganic non-metallic particles stabilized with a dispersant of the present invention having hydrophobic spacers, including but not limited to alkanes, for non-polar solvents and, hydrophilic polymer brushes, including but not limited to poly(ethylene glycol) for polar solvents may be used.

In another application, inorganic non-metallic particles, of the same or different materials, can be stabilized and crosslinked with dispersants having at least two anchor groups per linker group L or spacer. The anchor groups can be the same or different, covalently linked to a linker/spacer via connecting groups and e.g. through amide, ester urethane or azide bindings, as indicated in the formula below for a dispersant with a linker L or spacer S and two anchor groups, respectively, wherein A1, A2 are the anchor groups including the connecting groups, S designates the spacer and B1, B2 are the bindings.

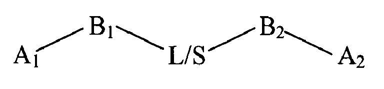

In this embodiment the group L/S may be a linker L or a spacer S. A spacer S will include at least one branch (not shown) for providing the needed distance between particles. In the case of a linear, optionally short, linker L the anchor groups A1 and/or A2 comprise a spacing group.

As already indicated above, dispersants for stabilizing and crosslinking inorganic non-metallic particles may comprise a spacer which has multiple branching points where more than two identical or different anchor groups are covalently linked to the spacer through e.g. amide, ester or urethane bindings. Optionally, one or multiple identical or different functional groups can also be linked to said spacers.

Inorganic non-metallic particles stabilized with dispersants of the present invention can also be used to form highly concentrated particle suspensions which are important for processing of inorganic non-metallic materials and/or to form highly concentrated particle suspensions for use as ferrofluids, and ferroelectric fluids and/or materials for data storage purposes, e.g. with magnetic particles.

As outlined above in detail, the dispersants of the present invention enable stabilization of inorganic non-metallic particles, especially ceramic particles, most notably contrast agents for biomedical imaging, such as magnetic resonance (and multi-modal) imaging. Examples of other applications are mesoporous and ceramic metamaterials and in the creation of structured smart materials for Lab-on-a-chip applications and as drug delivery and tracking vehicles and therapeutic agents for cancer therapy.

Due to the specific features of the inventive dispersants they adsorb irreversibly to the particle surface resulting in improved stability of the particles and thereby in a narrower particle size distribution and better control over particle properties such as size, surface charge and surface chemistry. The use of such particles as e.g. contrast agents results in superior performance.

Current state-of-the-art dispersants do not allow such stabilization since the bond formed to the particle surface is not strong enough under physiological conditions or generally for conceivable applications. The present invention allows to tailor (long) circulation times and also to perform controlled targeting or other tasks *in vivo* requiring a controlled number of stable functional ligands on the particle. It also allows any applications requiring temperatures up to the boiling point of water under physiological conditions.

The inventive dispersant is characterized by a cyclic ring (catechol) substituted with at least one and preferably one electron withdrawing group which allows electron exchange of the anchor with the inorganic non-metallic particles when binding to form a strong bond. This significant and qualitative difference to state of the art methods does not only enhance the performance of respectively stabilized ceramic particles, but also enables further applications that were previously not possible. The inventive anchor group attached to a polymer may be used for self-assembly of a core-shell particle, in particular particle, by the grafting to approach and is also applicable to functional particles for, e.g, biomedical imaging.

### Brief Description of the Drawings

The invention will be better understood and objects other than those set forth above will become apparent when consideration is given to the following detailed description thereof. Such description makes reference to the annexed drawings, wherein the Figures show:

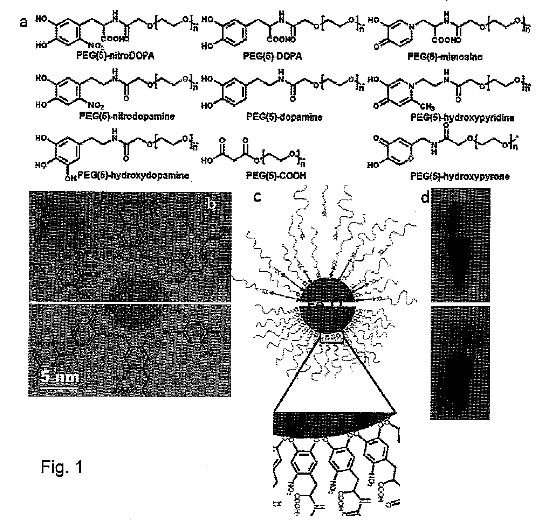

**Figure 1****.** Stabilization of iron oxide nanoparticles. a) Chemical structures of investigated dispersants with a degree of polymerization of n≈114 for PEG(M_{W} 5 kDa). b) High-resolution transmission electron micrograph (HRTEM) of iron oxide nanoparticles air-dried on a carbon supported Cu-grid. The hexagonal pattern of the inverse spinel structure of Fe₃O₄ oriented in the (010) direction is clearly visible proving the high crystallinity of these nanoparticles. Dispersants with different anchor groups have been grafted to as-synthesized iron oxide nanoparticles. c) Stabilization of iron oxide cores with PEG(M_{W} 5 kDa)-dopamine which adsorbed reversibly (top) and the irreversibly adsorbing PEG(M_{W} 5 kDa)-nitroDOPA (bottom). d) While PEG(M_{W} 5 kDa)-dopamine stabilized nanoparticles agglomerated and thus visibly precipitated (top), PEG(M_{W} 5 kDa)-nitroDOPA coated nanoparticles were stable even after being kept in HEPES for 20 h at 90 °C (bottom). The initial nanoparticle concentration was 1 mg iron oxide/ml thus, the color difference between PEG(M_{W} 5 kDa)-dopamine and PEG(M_{W} 5 kDa)-nitroDOPA stabilized nanoparticle dispersions is a result of the precipitation of PEG(M_{W} 5 kDa)-dopamine stabilized nanoparticles.

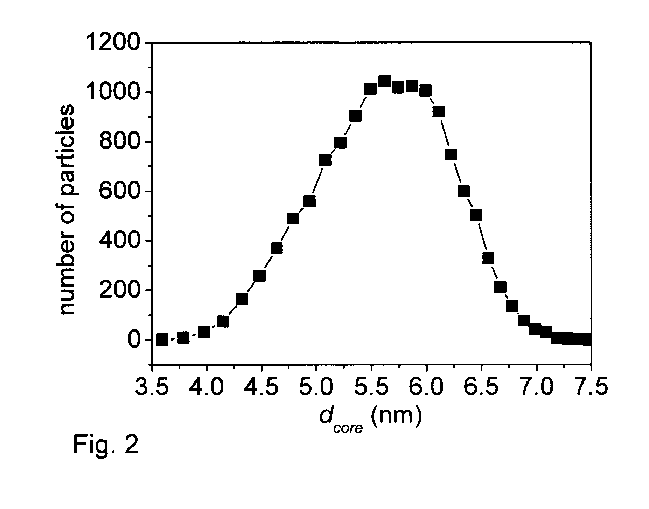

**Figure 2**. Iron oxide nanoparticle core size distribution. The iron oxide core size distribution was analyzed based on TEM micrographs of 13100 nanoparticles.
   Based on TEM micrographs, single domained iron oxide nanoparticle cores prepared by the microwave assisted non aqueous sol-gel route by heating them to 180 °C for 3 min were 6 ± 1 nm.

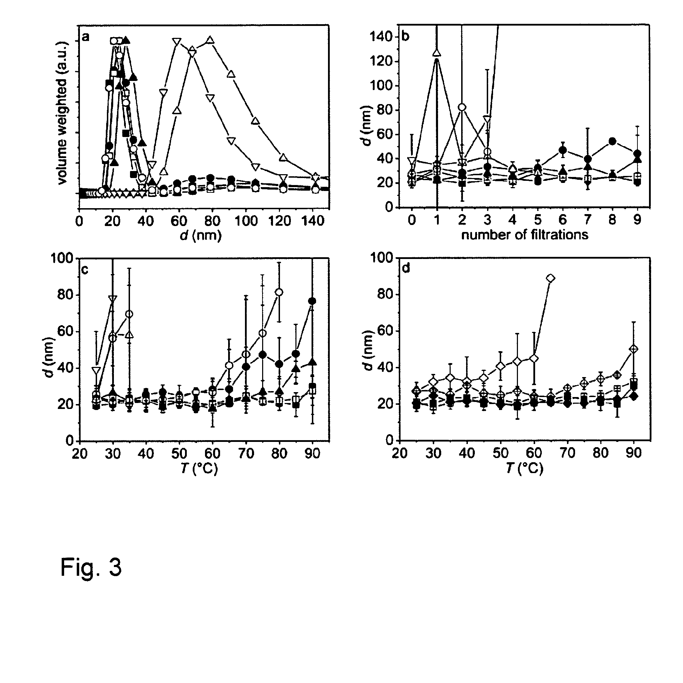

**Figure 3**. DLS measurements of individually stabilized iron oxide nanoparticles. Volume weighted diameters of iron oxide nanoparticles stabilized with PEG(M_{W} 5 kDa)-nitroDOPA (-■-), PEG(M_{W} 5 kDa)-nitrodopamine (-D-), PEG(M_{W} 5 kDa)-DOPA (-▲-), PEG(M_{W} 5 kDa)-dopamine (-△-), PEG(M_{W} 5 kDa)-mimosine (-●-), PEG(M_{W} 5 kDa)-hydroxypyridine (-○-) and PEG(M_{W} 5 kDa)-hydroxydopamine (-∇-) dispersed in HEPES at room temperature: a) as stabilized, b) as a function of the number of times particles have been filtered with the aim of probing the binding reversibility of the dispersants, and c) as a function of temperature. d) The particle stability at elevated temperature depended on the amount of dispersant added as shown for PEG(M_{W} 5 kDa)-nitroDOPA stabilized with 1 mg_{dispersant}/mg_{particle} (-◊-), 2 mg_{dispersant}/mg_{particle} (- 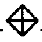 -), 4 mg_{dispersant}/mg_{particle} (- 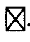 -), 6 mg_{dispersant}/mg_{particle} (-◆-) and 8 mg_{dispersant}/mg_{particle} (-■-). Standard deviations were calculated based on results measured on 3-6 identical, independent samples.

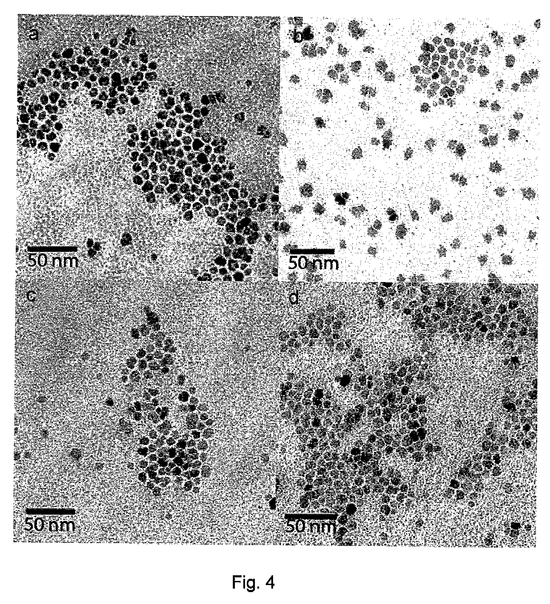

**Figure 4**. Overview TEM micrographs of iron oxide nanoparticles. Iron oxide nanoparticles stabilized with a) PEG(M_{W} 5 kDa)-nitroDOPA, b) PEG(M_{W} 5 kDa)-nitrodopamine, c) PEG(M_{W} 5 kDa)-mimosine and d) PEG(M_{W} 5 kDa)-DOPA air dried on a 10 nm thick carbon film supported Cu grid.

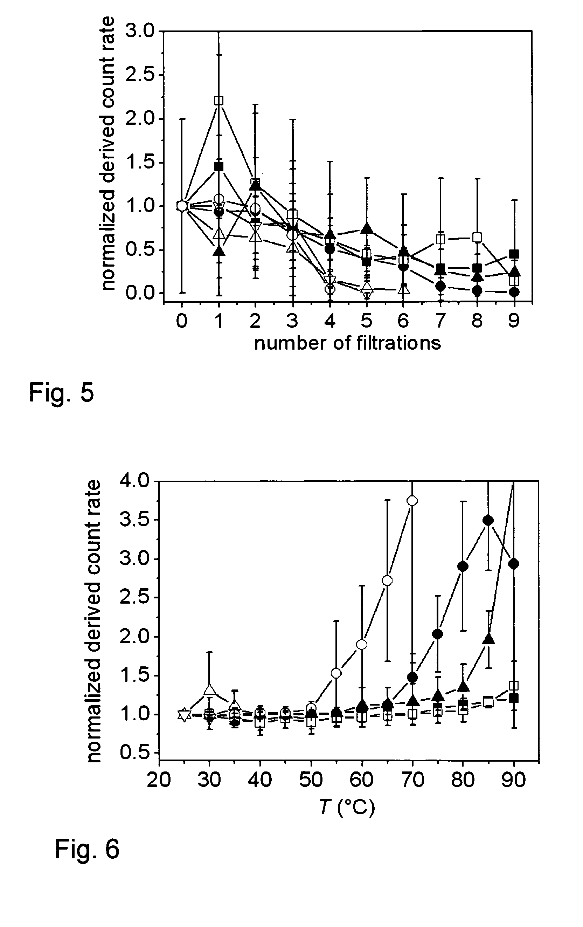

**Figure 5****.** Normalized count rates as a function of the number of filtrations investigated with DLS. The derived count rates are shown as a function of the number of filtrations for PEG(M_{W} 5 kDa)-nitroDOPA (-■-), PEG(M_{W} 5 kDa)-nitrodopamine (-□-), PEG(M_{W} 5 kDa)-DOPA (-▲-), PEG(M_{W} 5 kDa)-dopamine (△-), PEG(M_{W} 5 kDa)-mimosine (-●-), PEG(M_{W} 5 kDa)-hydroxypyridine (-○-) and PEG(M_{W} 5 kDa)-hydroxydopamine (-∇-) stabilized iron oxide nanoparticles dispersed in HEPES at room temperature as was measured by DLS. The derived count rate was normalized by the initial derived count rate. Statistics was done based on 3-6 identical independent samples.
**Figure 6****.** Normalized count rate dependence on the temperature measured by DLS. The derived count rates as a function of temperature for PEG(M_{W} 5 kDa)-nitroDOPA (-■-), PEG(M_{W} 5 kDa)-nitrodopamine (-□-), PEG(M_{W} 5 kDa)-DOPA (-▲-), PEG(M_{W} 5 kDa)-dopamine (-△-), PEG(M_{W} 5 kDa)-mimosine (-●-), PEG(M_{W} 5 kDa)-hydroxypyridine (-○-) and PEG(M_{W} 5 kDa)-hydroxydopamine (∇-) stabilized iron oxide nanoparticles dispersed in HEPES at room temperature. The derived count rate was normalized by the initial derived count rates. Statistics was done based on 3-6 independent identical samples.
   The temperature dependent irreversible aggregation measured by DLS as an increase in hydrodynamic diameter (Fig. 3c) could also be observed as a rapid increase in the derived count rate accompanied with an increase in the standard deviation (Fig. 3).

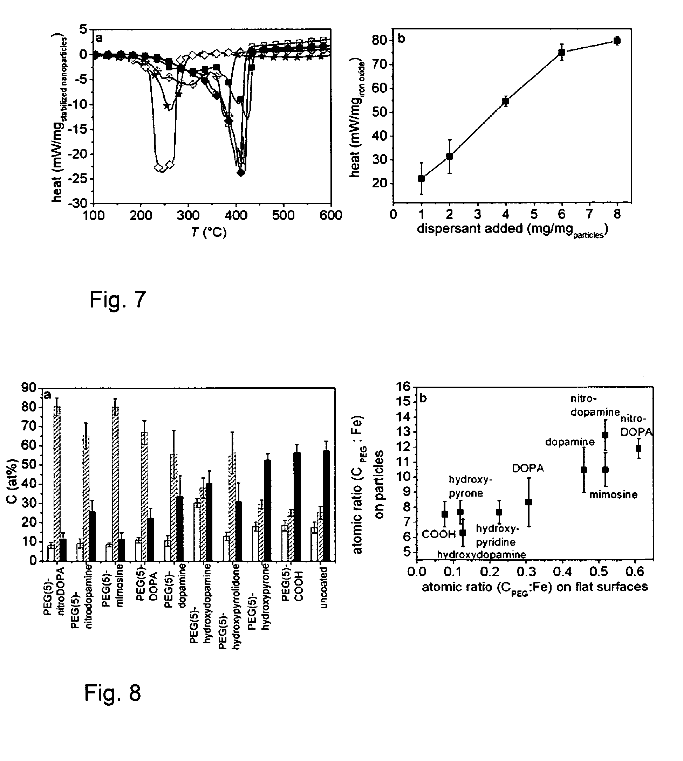

**Figure 7****.** TGA and DSC investigations of the optimal dispersant:iron oxide nanoparticle ratio. a) DSC graphs of PEG(M_{W} 5 kDa)-nitroDOPA stabilized iron oxide nanoparticles. Particles were stabilized with 1 mg_{dispersant}/mg_{particle} (-◊-), 2 mg_{dispersant}/mg_{particle} (- 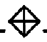 -), 4 mg_{dispersant}/mg_{particle} (- 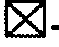 -), 6 mg_{dispersant}/mg_{particle} (-◆-), 8 mg_{dispersant}/mg_{particle} (-■-), respectively, and uncoated iron oxide nanoparticles (-★-). b) The heat dissipated during temperature induced decomposition of the organic part of iron oxide nanoparticles stabilized with different amounts of dispersants was measured by DSC. The dissipated heat was normalized to the iron oxide core weight.
**Figure 8**. XPS studies of dispersants adsorbed on flat 2D and nanoparticle surfaces. a) The atom percentage of C of PEG (C-O, white), aliphatic C (C-C, C-H, dashed) and OH-conjugated carbons (C-OH, black) of dispersants adsorbed on flat Fe₃O₄ surfaces was determined from XPS analysis. Statistics were taken for 3-6 independent, identical samples. b) The atomic ratio of C_{PEG}:Fe of dispersants adsorbed on flat Fe₃O₄ surfaces correlates well with that of dispersants adsorbed on iron oxide nanoparticles indicating that dispersant binding affinity is independent on surface curvature whereas the dispersant packing density is considerably higher on nanoparticles as compared to flat surfaces.

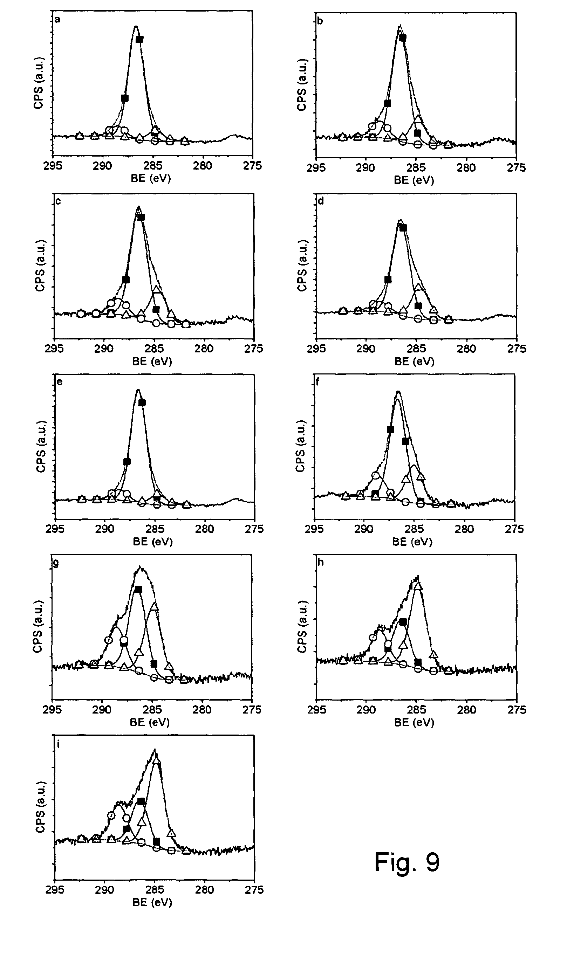

**Figure 9****.** XPS analysis of dispersants adsorbed on flat iron oxide surfaces. C1s peak of a) PEG(M_{W} 5 kDa)-nitroDOPA, b) PEG(M_{W} 5 kDa)-nitrodopamine, c) PEG(M_{W} 5 kDa)-DOPA, d) PEG(M_{W} 5 kDa)-dopamine, e) PEG(M_{W} 5 kDa)-mimosine, f) PEG(M_{W} 5 kDa)-hydroxypyridine, g) PEG(M_{W} 5 kDa)-hydroxydopamine, h) PEG(M_{W} 5 kDa)-hydroxypyrone and i) PEG(M_{W} 5 kDa)-COOH adsorbed on flat magnetron sputtered iron oxide surfaces. Three peaks have been fitted to the C1s peak. They correspond to PEG (-■-), aliphatic contaminations (-△-) and anchoring groups or COOH or CON conjugated contaminations (-○-)
   The raw XPS C 1s data shown in Fig. 9 and summarized in Fig. 8 show that next to the atomic ratio of C_{PEG}:Fe, the ratio of the C_{PEG} to C_{aliphatic} increased with increasing binding affinity of the anchor group and indicated that high affinity anchor groups could replace aliphatic contaminations as was already stated in the main text based on TGA and XPS analysis.

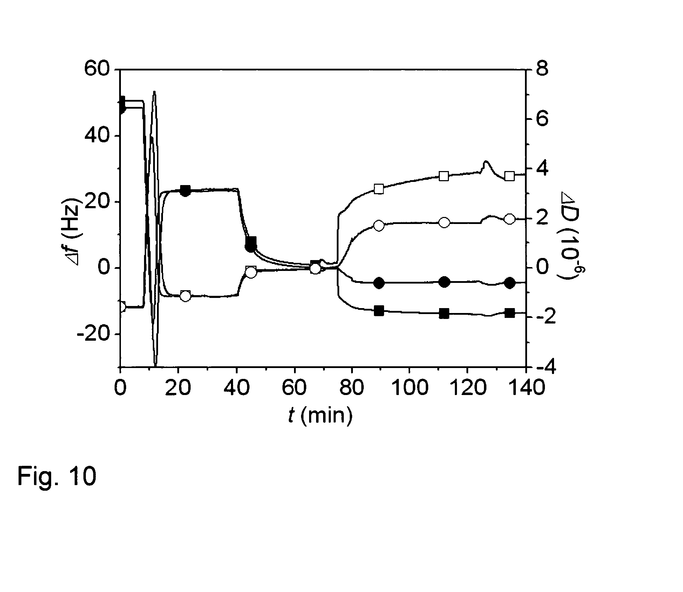

**Figure 10**: QCM-D measurements with biotin functionalized (-■-) and unfunctionalized (-●-) iron oxide nanoparticles. Neutravidin was immobilized on SiO₂ crystals through biotinylated supported lipid bilayers. Frequency (filled symbols) and dissipation (open symbols) decrease and increase respectively upon exposure of the neutravidin presenting supported lipid bilayer to iron oxide nanoparticles demonstrating adsorption of nanoparticles. Specific binding of the biotin functionalized particles is demonstrated by the much higher adsorbed amount of these nanoparticles compared to unfunctionalized nanoparticles.

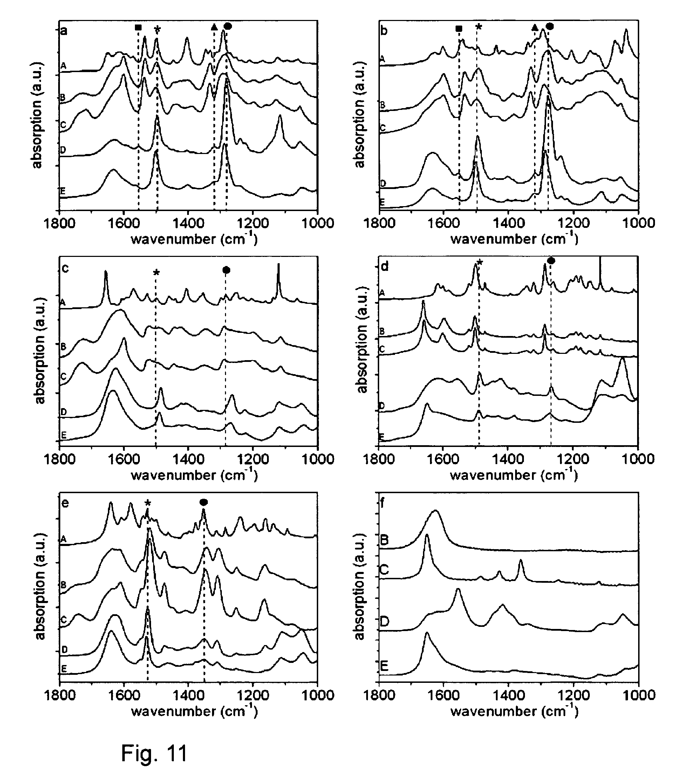

**Figure 11**: FTIR spectra of anchor/cation pairs. FTIR spectra of A) pure anchors were compared to those of anchors complexed with B) Fe²⁺ and C) Fe³⁺ ions in Millipore water at a molar ratio of anchor : iron ion=1:1 kept for 24 h at 50°C and those where anchors were adsorbed on D) Fe₃O₄ and E) TiO₂ nanoparticles. The investigated anchors were a) nitroDOPA, b) nitrodopamine, c) DOPA, d) dopamine and e) mimosine. f) Reference FTIR spectra of B) FeCl₂, C)FeCl₃, uncoated D) iron oxide and E) TiO₂ nanoparticles are shown. The most apparent vibrations measured on anchors adsorbed on Fe₃O₄ nanoparticles were the C-O stretch vibration (*), the C-C ring out of plane vibrations (●) and for nitrocatechols asymmetric (■) and symmetric (A) NO₂ vibrations.

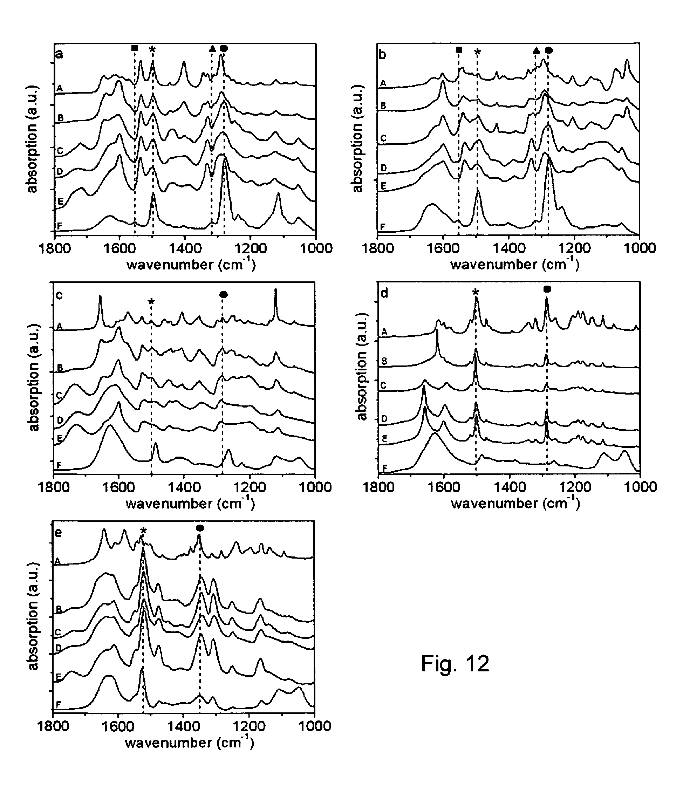

**Figure 12**: FTIR spectra of anchor/ion complexes. FTIR spectra of A) pure anchors, anchors complexed with B) Fe²⁺ and C) Fe³⁺ kept for 1 h at RT, D) Fe²⁺ and E) Fe³⁺ kept for 24 h at 50°C and F) anchors adsorbed on Fe₃O₄ nanoparticles for a) nitroDOPA, b) nitrodopamine, c) DOPA, d) dopamine and e) mimosine. The molar ratio of the anchor/iron complexes was always 1:1. The most pronounced vibrations were assigned to C-O stretch vibration (*), the C-C ring out of plane vibrations (●) and for nitrocatechols asymmetric (■) and symmetric (A) NO₂ vibrations.
   The C-O vibration peak of nitrocatechols complexed with Fe²⁺ was shifted to the position of the C-O vibration of nitrocatechols adsorbed on iron oxide nanoparticles after keeping these complexes at 50°C for 24 h. Furthermore, OH vibrations disappeared with time for nitrocatechols complexed with iron ions. However, while the relative intensity of DOPA complexed with iron ions decreased with increasing complexation time, spectra of dopamine complexed with iron ions resembled each other closely irrespective of complexation time and closely resemble those of uncomplexed dopamine. This indicates weak interactions of dopamine with iron ions. Furthermore, changes in FTIR spectra of mimosine/iron complexes with time were minor indicating high stability of mimosine/iron complexes.

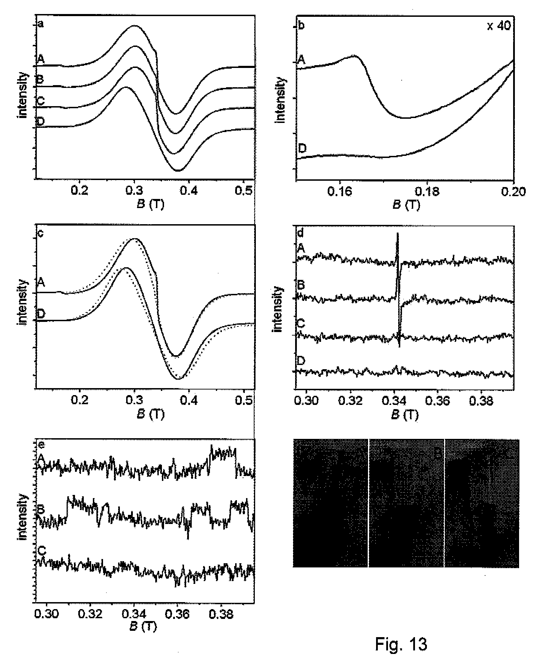

**Figure 13a)** Overview and b) close-up of EPR spectra of iron oxide nanoparticles stabilized with A) PEG(M_{W} 5 kDa)-nitroDOPA, B) PEG(M_{W} 5 kDa)-dopamine, C) PEG(M_{W} 5 kDa)-mimosine and D) uncoated iron oxide nanoparticles which still had residual precursors and physisorbed impurities on their surface. c) EPR spectra of A) PEG(M_{W} 5 kDa)-nitroDOPA and D) uncoated iron oxide nanoparticles before (solid) and after (dotted) dispersants were decomposed by subjecting nanoparticles to T>200°C. d) TiO₂ nanoparticles stabilized with A) PEG(M_{W} 5 kDa)-nitroDOPA, B) PEG(M_{W} 5 kDa)-dopamine, C) PEG(M_{W} 5 kDa)-mimosine and as a reference D) uncoated TiO₂ nanoparticles. EPR spectra were taken at room temperature. e) reference spectra of pure A) PEG(M_{W} 5 kDa)-nitroDOPA, B) PEG(M_{W} 5 kDa)-dopamine and C) PEG(M_{W} 5 kDa)-mimosine. f) TiO₂ nanoparticles stabilized with A) PEG(M_{W} 5 kDa)-nitroDOPA, B) PEG(M_{W} 5 kDa)-dopamine and C) PEG(M_{W} 5 kDa)-mimosine dispersed in Tris buffer at a concentration of 1 mg/ml.

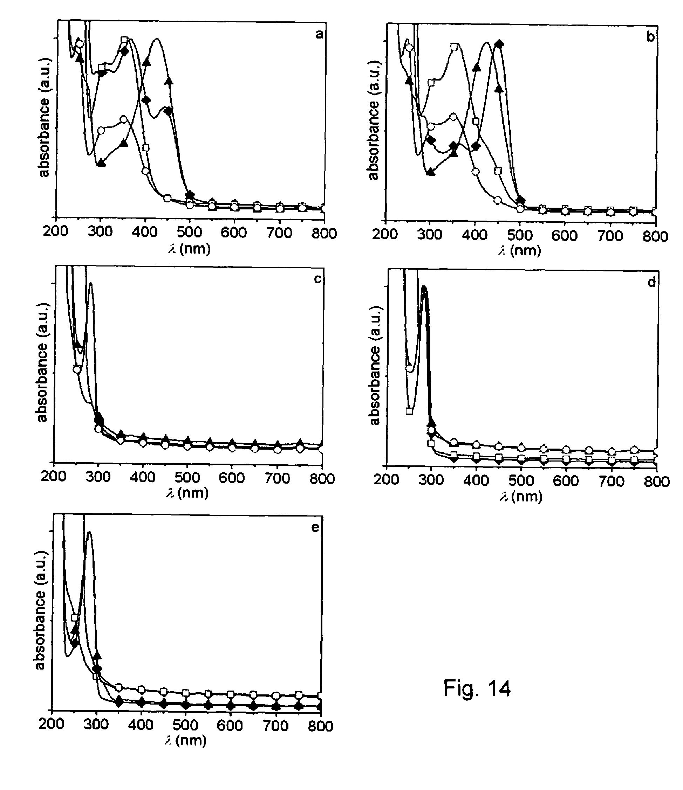

**Figure 14**: UV/VIS spectra of anchors. UV/Vis spectra of a) nitroDOPA, b) nitrodopamine, c) DOPA, d) dopamine and e) mimosine dispersed in Millipore water (-○-), Tris (-▲-), EtOH (-□-) and DMF (-◆-) respectively.
   π → π* transitions of benzene of nitrocatechols aliquotted in Millipore water were at markedly lower wavelengths compared to those of catechols and mimosine. However, only a small shoulder was visible for the π → π* transition of nitrocatechols if aliquotted in Tris in contrast to catechols and mimosine, where a clear π → π* transition peak was apparent. Moreover, the HOMO→LUMO transition of nitrocatechols was more pronounced and at higher wavelengths if dispersed in Tris compared to Millipore water. This indicates a stronger electron shift from the catechol ring to the nitro group with increasing pH.

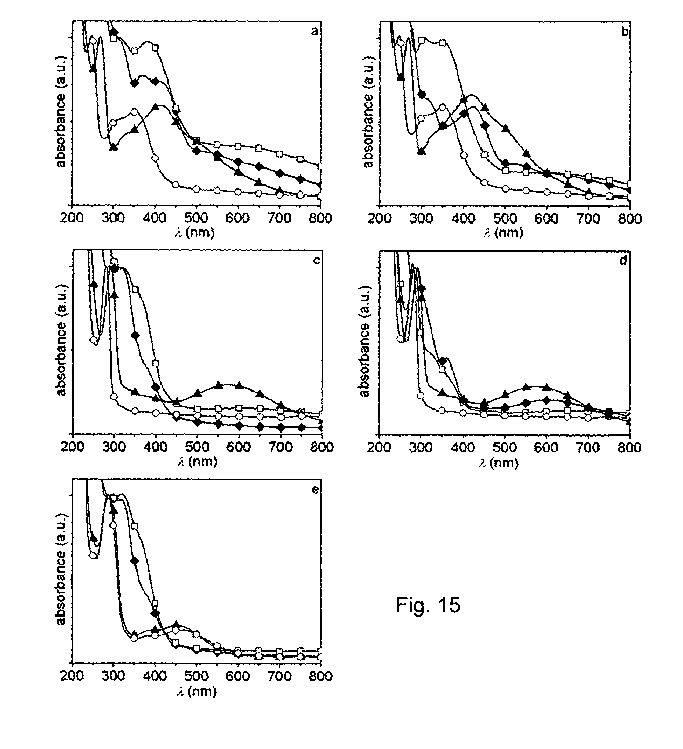

**Figure 15****:** UVNIS spectra of anchor/Fe²⁺ complexes. UV/VIS spectra of a) nitroDOPA, b) nitrodopamine, c) DOPA, d) dopamine and e) mimosine complexed with Fe²⁺ dispersed in Millipore water (-○-), Tris (-▲-), EtOH (-□-) and DMF (-◆-) respectively The molar ratio of anchors : iron ions was 1:1. Complexes were kept at RT for 24 h before UVNIS spectra were measured.

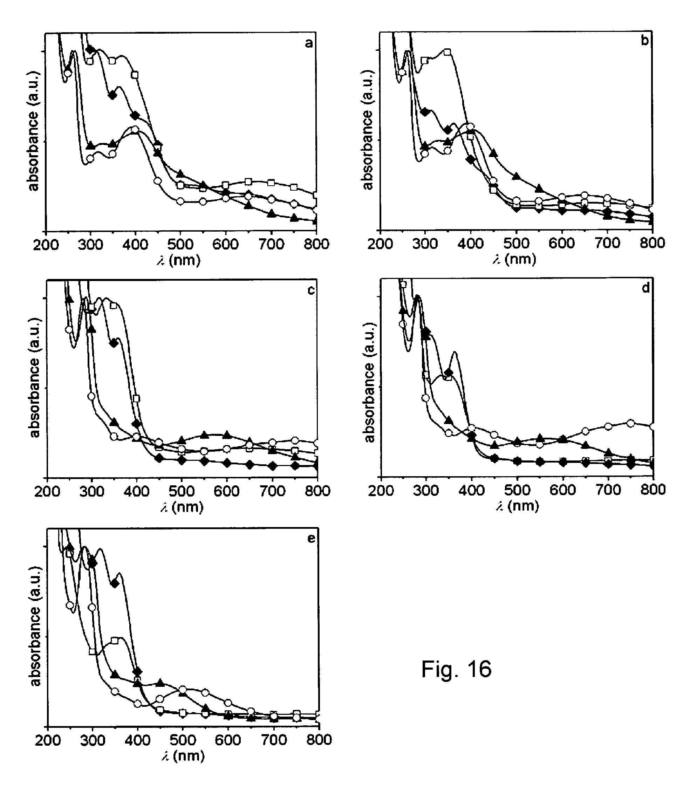

**Figure 16****:** UVNIS spectra of anchor/Fe³⁺ complexes. UVNIS spectra of a) nitroDOPA, b) nitrodopamine, c) DOPA, d) dopamine and e) mimosine complexed with Fe³⁺ in Millipore water (-○-), Tris (-▲-), EtOH (-□-) and DMF (◆-) respectively. The molar ratio of anchors : iron ions was 1:1. Complexes were kept at RT for 24 h before UVNIS spectra were measured.

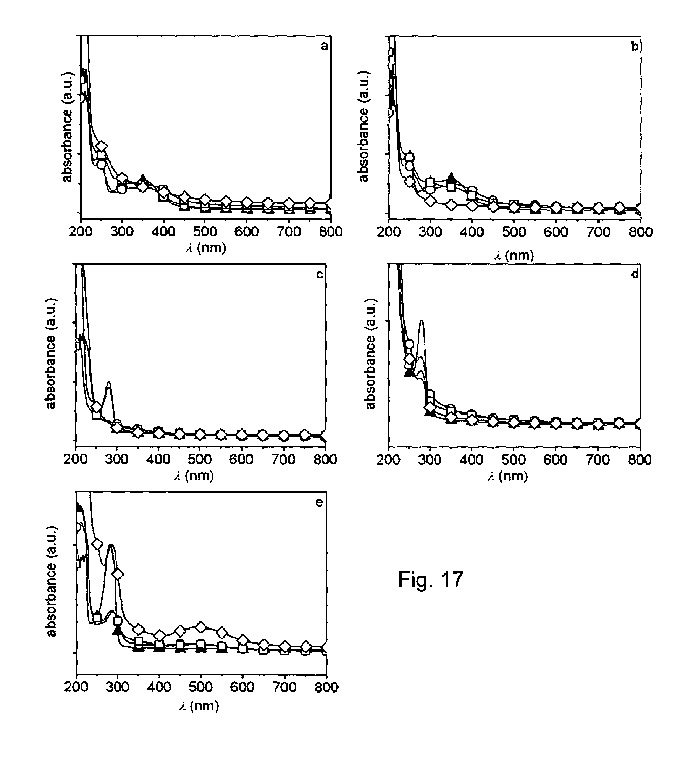

**Figure 17****:** UVNIS spectra of supernatants of iron oxide nanoparticles. UVNIS spectra of the supernatant of a) PEG(M_{W} 5 kDa)-nitroDOPA, b) PEG(M_{W} 5 kDa)-nitrodopamine, c) PEG(M_{W} 5 kDa)-DOPA, d) PEG(M_{W} 5 kDa)-dopamine and e) PEG(M_{W} 5 kDa)-mimosine stabilized iron oxide nanoparticles (-◊-) and as a comparison the respective anchors complexed with Fe²⁺ (-○-) and Fe³⁺ (□-) and uncomplexed (-▲-). All these solutions were Millipore water based and the molar ratio of anchors to iron ions was 1:1.
   A comparison of UVNIS spectra of the supernatant of PEG(M_{W} 5 kDa)-mimosine stabilized iron oxide nanoparticles with those of mimosine/iron complexes and uncomplexed mimosine reveals the presence of PEG(M_{W} 5 kDa)-mimosine/Fe³⁺ complexes in the supernatant in contrast to the other dispersants investigated. Thus there is gradual dissolution of PEG(M_{W} 5 kDa)-mimosine stabilized iron oxide nanoparticles induced by the strong complexation of mimosine to iron.

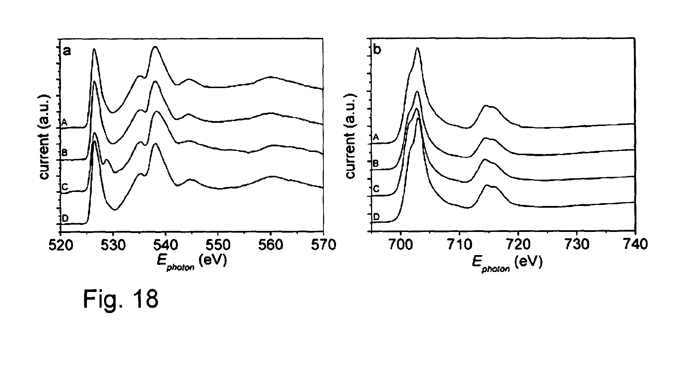

**Figure 18****:** NEXAFS spectra of anchors adsorbed on flat iron oxide surfaces. a) O K-edge and b) Fe L-edge NEXAFS spectra of A) DOPA, B) dopamine, C) mimosine and D) uncoated iron oxide surfaces taken at 90° incidence angle.

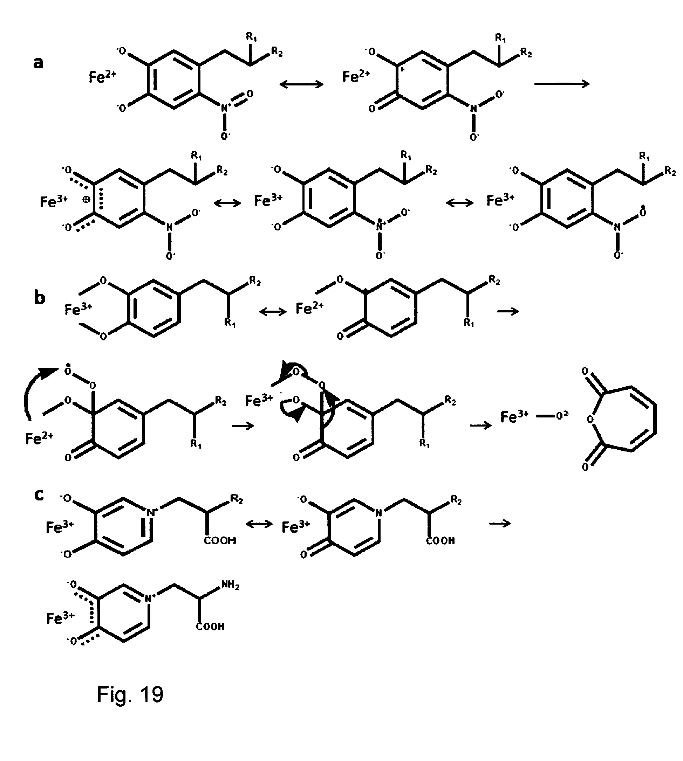

**Figure 19****:** Reactions of anchors with iron. A) Redox reaction of nitrocatechol complexed with Fe²⁺ ions. B) Iron catalyzed catechol degradation. C) mimosine complexed with Fe³⁺. R1= H for dopamine and COOH for DOPA whereas R2 were amines for pure feet and PEG for dispersants used to stabilize nanoparticles

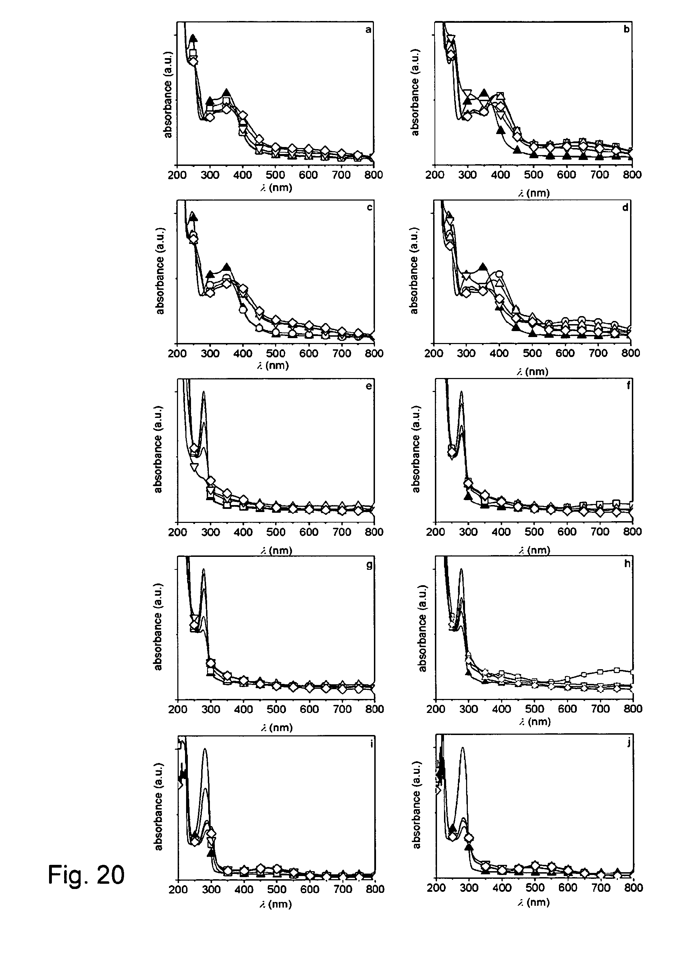

**Figure 20****.** Time and temperature dependence of anchor/iron complexes. UVNIS spectra of a) nitroDOPA/Fe²⁺, b) nitroDOPA/Fe³⁺, c) nitrodopamine/Fe²⁺, d) nitrodopamine/Fe³⁺, e) DOPA/Fe²⁺, f) DOPA/Fe³⁺, g) dopamine/Fe²⁺, h) dopamine/Fe³⁺, i) mimosine/Fe²⁺, j) mimosine/Fe³⁺ complexes dispersed in Millipore water after 1 h (-□-), 1 d (-△-) and 8 d (-◊-) if kept at RT and after 1 d kept at 50°C (-∇-) respectively. As a reference, uncomplexed anchors (-▲-) are shown.
   While UV/VIS spectra of nitrocatechols and mimosine complexed with iron ions did not change significantly if kept at RT even after 8 d, π → π* transitions of complexed catechols disappeared if kept for 8 d at room temperature indicating a catechol degradation well in agreement with FTIR and UV/VIS results. Furthermore, catechol degradation was accelerated if kept at 50°C, where no π → π* transition was seen after 1 d. The significant broadening of the HOMO→LUMO transition of nitrocatechols upon complexation happened within the 1^{st} hour after complexation and did not change with time any more indicating that these complexes are stable.

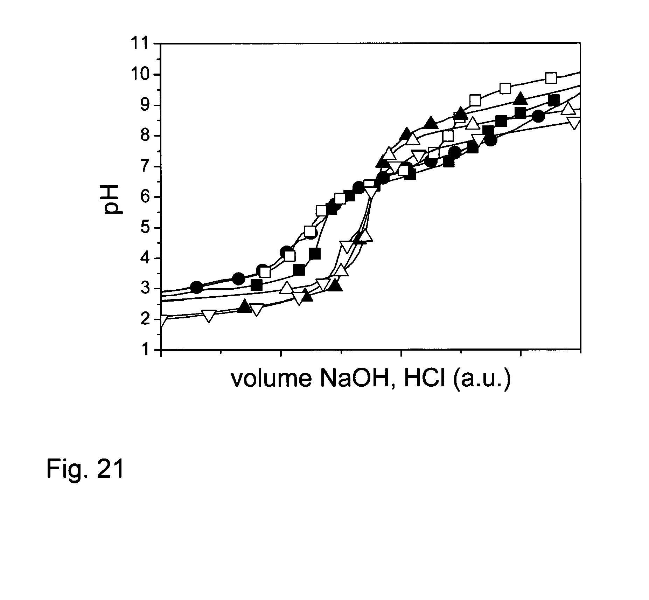

**Figure 21****.** pKₐ determination. Titration curves of nitroDOPA (-■), nitrodopamine (-□-), mimosine (-●-), DOPA (-▲-), dopamine (-△-) and hydroxydopamine (-∇-). These titration curves were measured in Millipore water 150 mM NaCl based solutions
   One of the pKₐ values of nitroDOPA, nitrodopamine and mimosine was found to be around 6.5, close to the PZC of iron oxide (pKₐ=6.7), while dopamine, hydroxydopamine and DOPA have their pKₐ values above pH=9 (Fig. 21).

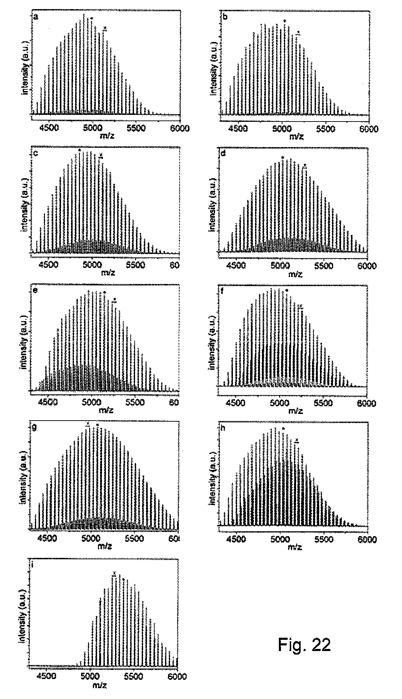

**Figure 22****.** Mass spectroscopy analysis of dispersants. a) PEG(M_{W} 5 kDa)-nitroDOPA, b) PEG(M_{W} 5 kDa)-nitrodopamine, c) PEG(M_{W} 5 kDa)-mimosine, d) PEG(M_{W} 5 kDa)-DOPA, e) PEG(M_{W} 5 kDa)-dopamine, f) PEG(M_{W} 5 kDa)-hydroxypyridine, g) PEG(M_{W} 5 kDa)-hydroxydopamine, h) PEG(M_{W} 5 kDa)-hydroxypyrone, i) PEG(M_{W} 5 kDa)-COOH. m/z values of peaks indicated with a star are reported in Tab. S3. Δm/z (x) is 44 Da, corresponding to the ethylene glycol repeat unit.

### Modes for Carrying Out the Invention

Some embodiments of the present invention are further investigated below and compared with embodiments using anchor groups known from the state of the art.

The following Experiments are performed using the materials and analytical methods further described below.

*Materials:* Fe(ac)₂ (batch 517933, Lot 03901JJ), mimosine, dopamine, diethylether, 4-(2-hydroxyethyl)-1-piperazineethanesulfonic acid (HEPES), sodium tetraborate decahydrate (borate buffer), D₂O, ethanol and N,N-Dimethylformamide (DMF) were purchased from Sigma-Aldrich, DOPA, hydroxydopamine, benzylalcohol and Chloroform from Acros and PEG(M_{W} 5 kDa)-NHS and PEG(M_{W} 5 kDa)-COOH from Jemkem, NaCl from J. T. Baker and ethanol from Scharlau.

*Anchor groups:* Nitrodopamine and nitroDOPA were synthesised by adding 20% sulphuric acid to an in ice cooled water based solution containing sodium nitrite and dopamine or DOPA respectively. Hydroxypyridine was synthesised starting from kojic acid by replacing the aliphatic alcohol by chlorine followed by a removal of chlorine. The aromatic alcohol was protected with benzyl before the aromatic oxygen was substituted with ethylenediamine. Hydroxypyrone was synthesised starting from kojic acid. The aliphatic alcohol was replaced by chlorine and subsequently by azides before it was converted to konjic amine.

*Dispersants:* Poly(ethylene glycol)-N-hydroxy succinimide ester, where the poly(ethylene glycol) had a molecular weight of 5 kDa, (PEG(M_{W} 5 kDa)-NHS was coupled to the different anchor groups. Briefly, PEG(M_{W} 5 kDa)-NHS (1 mol equ) was dissolved in borate buffer (pH=8.4) (20 ml) before the respective anchor group (0.8 mol equ) was added. After 24 h reaction at room temperature under constant magnetic stirring, the solution was acidified with HCl. It was extracted with chloroform before it was precipitated in diethylether and vacuum dried. Dispersants were analyzed with matrix assisted laser desorption/ionisation time of flight (MALDI-TOF) analysis (Fig. 22 and Tab. 5), microelemental analysis (Tab. 6), ¹Hand ¹³C-NMR respectively.

*Iron oxide nanoparticles:* Iron oxide nanoparticles were synthesized in a microwave assisted non-aqueous sol-gel route. Briefly, Fe(ac)₂ (173 mg) was dissolved in benzylalcohol (5 ml) and heated for 3 min at 180°C in a microwave (Discover S-class, CEM, NC, USA). These nanoparticles were washed once with ethanol (10 ml) before they were re-dispersed in ethanol (10 ml) resulting in an iron oxide nanoparticle concentration of approximately 10 mg/ml. Washed iron oxide nanoparticles were kept at 4°C for 2-6 h before these particles (1 mg) were added to DMF or ethanol (1 ml) containing dispersants (unless stated otherwise 6-8 mg) respectively. This suspension was kept at 50°C for 24 h under constant mechanical stirring at 500 rpm (Thermomixer comfort, Vaudaux-Eppendorf, Switzerland). Excessive dispersants were removed by 24 h dialysis against Millipore water (*R*=18.2 Ω, TAC<6 ppb) using dialysis membranes with a cut-off of 14-25 kDa (Spectra/Por dialysis membrane, spectrum labs, Netherlands) before so-stabilized iron oxide nanoparticles were freeze-dried (freeze dryer ALPHA 1-2 / LDplus, Kuhner LabEquip, Switzerland).

*DLS:* DLS experiments were run on a Zetasizer Nano ZS (Malvern, UK) in the 173° backscattering mode. Because intensity weighted diameters scale with r⁶ and thus large particles are predominant, volume weighted data which scale with r³ were analyzed using the multiple narrow modes evaluation incorporated into the Malvern software. Temperature dependent DLS experiments have been performed in HEPES at an iron oxide nanoparticle concentration of 200 µg/ml. Solutions were filtered with 200 nm Minisart syringe filters (Sartorius, Germany) prior to DLS analysis. These suspensions were heated from 25 to 90°C in 5°C steps with 10 min equilibration time before the respective DLS experiment. The dispersant binding reversibility was tested on iron oxide nanoparticle HEPES solutions (500 µg/ml) at room temperature. Solutions were repeatedly filtered using Microcon filters with a cut-off of 30 kDa (Millipore, MA, USA) by centrifuging these solutions at 13 rpm for 20 min (MiniSpin, Vaudaux Eppendorf, Switzerland). After filtration, the filter cake was re-suspended in HEPES (0.5 ml) and equilibrated for 24 h at room temperature before the next DLS experiment was performed.

*TGA*/*DSC:* Stabilized iron oxide nanoparticles (5 mg) were re-dispersed in Millipore water (500 µl) for 24 h before they were filtered with Microcon centrifuge filters with a cut-off of 30 kDa. The filter cake re-dispersed in Millipore water (0.5 ml) was freeze-dried. Alternatively, stabilized nanoparticles were dialyzed for 24 h against Millipore water using dialysis tubes with a cut-off of 25 kDa before these nanoparticles were freeze-dried. So-prepared nanoparticles (3-5 mg per run) were analyzed on a NETZSCH STA 449 C Jupiter, which for some experiments was coupled to a NETZSCH QMS 403 Aeolos MS (NETZSCH Geraetebau GmbH, Austria). Samples were heated from 35°C to 600 °C at 10°C/min using a flow rate of 47.4 sccm Ar and 12.6 sccm O₂.

*TEM:* Transmission electron micrographs were taken on a Philips CM12 microscope operated at 100 kV and on a HRTEM Philips CM30 operated at 300 kV. Iron oxide nanoparticles suspended in Millipore water were dried on a 10 nm carbon coated 400 mesh Cu-grid. Diffraction patterns were analyzed using jems ems java version 3.2927U2008 software (P. Stadelmann, EPFL, Switzerland).

*XPS:* Stabilized iron oxide nanoparticles (5 mg) were re-dispersed in Millipore water (0.5 ml) for 24 h before they were centrifuge filtered with Microcon filters with a cut-off of 30 kDa. Filter cakes were re-dispersed in Millipore water, freeze-dried and fixed onto vacuum compatible double sided sticky tapes. XPS was performed on a Sigma Probe (Thermo Scientific, MA, USA), using an Al K_{α} source operated at 200 W. Photoelectrons were detected with a hemispherical analyser at a pass energy of 25 eV at 90° take off angle. Data were analyzed using the CasaXPS software (CasaXPS software Version 2.3.15dev52, Software Ldt, UK). A Shirley background was subtracted before the peak areas were integrated and corrected for the cross section using the Scofield factors, inelastic mean free path, attenuation length and the energy dependent transmission function. The dispersant layer thickness was calculated by relating the intensities of the Fe^{2p} and Fe^{3p} peaks. The atomic C:Fe ratio of nanoparticles was quantified by considering the 3D shape of nanoparticles . The dispersant packing density on flat surfaces was calculated based on the dispersant thickness measured by XPS and a PEG density of 1.1 g/cm³.

*TiO₂ nanoparticles:* TiO₂ nanoparticle were synthesized by slowly adding 1 ml TiCl₄ to 5 ml ethanol followed by the addition of 20 ml benzylalcohol. This solution was magnetically stirred at 80°C for 8 h before the suspension was precipitated in diethyl ether and washed once with ethanol. The resulting TiO₂ nanoparticles were re-suspended in ethanol before they were coated with anchor groups as described for the iron oxide nanoparticles.

*Flat Fe₃O₄ substrates:* Thin (<10nm) Fe₃O₄ films have been magnetron sputtered (PVD products, MA, USA) onto one side polished 10×10 mm² Si wafers (Silicon materials, Germany). Metallic Fe targets (purity 99.9%, Kurt J. Lesker, UK) were reactive sputtered at P=200 W for 10 min at *p*=5 mtorr. The influence of *p(O₂)* during reactive sputtering on the resulting iron oxide stoichiometry was investigated with XPS. It was found that an atmosphere consisting of 20 vol% O₂ and 80 vol% Ar resulted in thin Fe₃O₄ films.

*Adsorption on flat Fe₃O₄ surfaces:* As sputtered Fe₃O₄ substrates were cleaned for 30 min by UV/ozone (UV clean Model 135500, BEOKEL, PA, USA) before they were inserted into a DMF based solution containing dispersants (100 µg/ml). Dispersants were adsorbed for 24 h at 50°C before the substrates were thoroughly rinsed with Millipore water and dried with N₂.

*pKₐ determination:* 2 mM of the respective anchoring group was dissolved in Millipore water containing 150 mM NaCl. These solutions were titrated with 0.1 M HCl and NaOH respectively using a SevenMulti pH meter (Mettler Toledo, Switzerland).

*MALDI-TOF:* MALDI-TOF was measured by the MS service of the organic chemistry laboratory at ETH Zurich. It was done on a Bruker Daltonics Ul-traflex II equipped with a YAG Laser (pulse rate= 50 ps). The matrix consisted of a mixture of DCTB+Na of 1:10:1.

*NMR:* ¹H-NMR and ¹³C-NMR were measured on a Bruker 500 MHz NMR spectrometer in D₂O.

*Microelement analysis:* Microelement analysis was performed in the Microlaboratory of organic chemistry, ETH Zurich.

*Complexation:* 0.505 µmol anchors were added to 0.505 µmol FeCl₂ and FeCl₃ respectively which was dissolved in 1 ml Millipore water (R=18.2 Ω, TAC<6 ppb) or 1 ml 10 mM Tris(hydroxymethyl)aminomethane containing 150 mM NaCl (Tris) respectively. Unless stated otherwise, these solutions were left at RT for 1 h before they were analyzed.

*FTIR spectroscopy:* FTIR was done on KBr tablets where the weight ratio of KBR: sample was ≈100:1. FTIR spectra were recorded from 400 to 4000 cm⁻¹ at a resolution of 4 cm⁻¹ at room temperature at a pressure of <2 mbar on a Bruker IFS 66v instrument using a DTGS detector. An average of 32 spectra were taken.

*EPR:* 2-3 mg PEG(M_{W} 5 kDa)-nitroDOPA and PEG(M_{W} 5 kDa)-dopamine stabilized and as a reference unstabilized iron oxide nanoparticles were analyzed at a time. EPR measurements were done on a Bruker EMX spectrometer at a frequency of 9.86 GHz at room temperature. An average of 4 scans was taken.

*UV*/*VIS spectroscopy:* UV/VIS spectroscopy was performed on 0.505 µM solutions. Spectra were recorded on a Cary 1E UV/VIS spectrometer (Va-rian) between 200 and 800 nm.

*NEXAFS measurements:* NEXAFS spectra have been acquired at the PolLux beamline at PSI Villigen (Switzerland) using the total electron yield mode (TEY) under an X-ray incidence angle of 90° where the pressure was kept below 2×10⁻⁴ mbar. Statistics was done on two identical independent samples.

Eight different catechol derivatives were selected to shed light on the design principles for optimized anchors for dispersants to stabilize iron oxide nanoparticles. Poly(ethylene glycol) (PEG) with a molecular weight of 5 kDa (PEG(M_{W} 5 kDa)) was coupled to each of the different anchor groups to yield dispersants of suitable size for SPION stabilization.

In the scope of the present invention it could be shown that despite the numerous applications of dopamine and DOPA as high affinity anchor group for iron oxide nanoparticle stabilization there can be designed anchors which, if coupled to a spacing group such as PEG, result in greatly enhanced iron oxide nanoparticle stability. Furthermore, it could be shown that conventional dopamine-based dispersants fail to stabilize iron oxide nanoparticles at elevated temperature and that they desorb upon dilution of the iron oxide nanoparticle solution under physiologic conditions in strong contrast to the dispersants of the present invention some of which are further investigated here. This failure of dopamine-based dispersants to stabilize iron oxide nanoparticles under relevant conditions severely limits their applicability in the biomedical field where stability at body temperature, in diluted suspensions and physiological ion concentrations is key. Thus, there are obvious advantages of replacing dopamine by the inventive anchor groups, which yield improved nanoparticle dispersion stability.

### Example 1: Iron oxide nanoparticle stability

The nine different PEG-based dispersants used for the present investigations are shown in Fig. 1a. Among the different anchor groups, in addition to dopamine, also L-DOPA and hydroxydopamine have been reported to lead to good iron oxide nanoparticle stability[1,5]. Furthermore, PEG(M_{W} 5 kDa)-COOH was investigated because dopamine and DOPA, and other investigated catechol derivatives chemically differ only in one carboxy group, motivating the inventors to investigate the role of carboxylates in iron oxide binding of these anchors. Single domain magnetite (Fe₃O₄) cores prepared by the microwave assisted non-aqueous sol-gel route had a diameter of 6 ± 1 nm and were stabilized with the different dispersants through a simple grafting to method (Fig. 1 and Fig. 2). As-synthesized iron oxide nanoparticle cores were added to a N,N-dimethylformamide (DMF) or alternatively ethanol (EtOH) based dispersant solution. Dispersants were adsorbed at 50°C for 24 h before excessive dispersants were removed and DMF or EtOH respectively was exchanged by Millipore water through 24 h dialysis against Millipore water. Stabilized iron oxide nanoparticles were freeze-dried and re-dispersed in the desired media at appropriate concentrations.

PEG(M_{W} 5 kDa)-hydroxypyrone and PEG(M_{W} 5 kDa)-COOH stabilized iron oxide nanoparticles did not pass filters with a cut-off of 200 nm, which demonstrated that they instantaneously formed agglomerates larger than 200 nm in diameter. Consequently, no reliable dynamic light scattering (DLS) could be performed on them.

The average volume-weighted hydrodynamic diameter of iron oxide nanoparticles individually stabilized with PEG(M_{W} 5 kDa)-nitroDOPA, PEG(M_{W} 5 kDa)-nitrodopamine, PEG(M_{W} 5 kDa)-mimosine, PEG(M_{W} 5 kDa)-DOPA, PEG(M_{W} 5 kDa)-dopamine, PEG(M_{W} 5 kDa)-hydroxydopamine and PEG(M_{W} 5 kDa)-hydroxypyridine respectively and dispersed in 4-(2-hydroxyethyl)-1-piperazineethanesulfonic acid containing 150 mM NaCl (HEPES) at room temperature was 23±2 nm (Fig. 3a). So-stabilized iron oxide nanoparticles could be freeze-dried, stored as a powder for at least 3 months or alternatively re-dispersed in Millipore water and stored for at least 4 months without noticeable change in particle stability and hydrodynamic diameter after re-dispersing and diluting them in HEPES. A clear separation of the cores and the absence of aggregates of iron oxide nanoparticles stabilized with PEG(M_{W} 5 kDa)-nitroDOPA, PEG(M_{W} 5 kDa)-nitrodopamine, PEG(M_{W} 5 kDa)-mimosine and PEG(M_{W} 5 kDa)-DOPA supports the DLS result of individually stabilized iron oxide nanoparticles visualized by TEM (Fig. 4).

The binding reversibility of these dispersants was tested by successive filtrations and re-dispersions of the resulting iron oxide nanoparticle filter cakes in HEPES

Nanoparticles stabilized with low (dopamine, hydroxydopamine, hydroxypyridine, hydroxypyrone and carboxyl), intermediate (DOPA) and too high (mimosine, causing dissolution of the particles) affinity anchors agglomerated within 0-3 and 5-7 filtrations respectively. Nanoparticles stabilized with high affinity (nitroDOPA and nitrodopamine) anchors, remained stable for more than 9 filtrations (Fig. 3b and Fig. 5). This indicated essentially irreversible binding of nitroDOPA and nitrodopamine to iron oxide nanoparticles under physiologic conditions, in stark contrast to other investigated anchor groups. The desorption rate of intermediate affinity dispersants, however, was significantly lower compared to that of low affinity dispersants, which indicated that although the binding was reversible the affinity of these intermediate dispersants towards iron oxide is much higher.

While the derived count rate (see Fig. 6) increased if particles agglomerated due to stronger scattering of larger particles (the intensity of scattered light scales with r⁶), it decreased upon sedimentation of those agglomerates. Because iron oxide nanoparticle suspensions analyzed here had been equilibrated for 24 h after each filtration before DLS was measured, larger agglomerates could sediment and thus were not analyzed by DLS any more. This resulted in a marked decrease in the derived count rates.

Even though the standard deviations were large, the normalized derived count rates of PEG(M_{W} 5 kDa)-dopamine, PEG(M_{W} 5 kDa)-hydroxydopamine and PEG(M_{W} 5 kDa)-hydroxypyridine stabilized iron oxide nanoparticles decreased faster compared to particles stabilized with PEG(M_{W} 5 kDa)-nitroDOPA, PEG(M_{W} 5 kDa)-nitrodopamine, PEG(M_{W} 5 kDa)-DOPA and PEG(M_{W} 5 kDa)-mimosine, well in agreement with the change in hydrodynamic diameter which increased faster for iron oxide nanoparticles stabilized with the former dispersants. The change in count rate is a very good measure of agglomeration and sedimentation reducing the artifact of looking only at the change of the hydrodynamic diameter of the particles remaining in solution.

Depending on the dispersant, individually stabilized iron oxide nanoparticles dispersed in HEPES started to agglomerate upon heating. While iron oxide nanoparticles stabilized with PEG(M_{W} 5 kDa)-nitroDOPA and PEG(M_{W} 5 kDa)-nitrodopamine could be repeatedly heated up to 90 °C without noticeable agglomeration or changes in the derived count rates as measured by DLS, iron oxide nanoparticles stabilized with low affinity dispersants started to irreversibly agglomerate already below body temperature. Particles stabilized with intermediate and too high affinity dispersants agglomerated between 60 and 80 °C, as indicated by an increase in the hydrodynamic diameter (Fig. 3c) and an increase followed by a decrease in the derived count rate due to particle agglomeration and sedimentation, respectively (see Fig. 6). However, even for irreversibly binding dispersants, a concentration of at least 6 mg_{dispersants}/mg_{nanoparticle} was needed to completely prevent nanoparticle aggregation up to 90°C (Fig. 3d).

### Example 2: Dispersant packing density

The dispersant packing density on iron oxide nanoparticles was determined by thermogravimetric analysis (TGA) between 200 and 400°C (see Table 1).

**Table 1. Mass losses measured by TGA between 200 and 450 °C for iron oxide nanoparticles stabilized with 8 mg of different dispersants per mg nanoparticle.**

| | mass loss (wt%) |
|---|---|
| PEG(M_{W} 5 kDa)-mimosine | 80±5 |
| PEG(50)-mimosine heated to 90°C prior to analysis | 84±4 |
| PEG(M_{W} 5 kDa)-nitroDOPA | 83±1 |
| PEG(M_{W} 5 kDa)-nitroDOPA heated to 90°C prior to analysis | 85 |
| PEG(M_{W} 5 kDa)-nitrodopamine | 82±1 |
| PEG(M_{W} 5 kDa)-DOPA | 79±7 |
| PEG(M_{W} 5 kDa)-dopamine | 79±4 |
| PEG(M_{W} 5 kDa)-dopamine heated to 90°C prior to analysis | 51 |
| PEG(M_{W} 5 kDa)-hydroxydopamine | 74±6 |
| PEG(M_{W} 5 kDa)-hydroxypyridine | 75±6 |
| PEG(M_{W} 5 kDa)-hydroxypyrone | 75±2 |
| PEG(M_{W} 5 kDa)-COOH | 69±10 |
| uncoated | 15±8 |

Related to the packing density, the atomic C_{PEG}:Fe ratio was investigated by X-ray photoelectron spectroscopy (XPS) (Tab. 2 and Fig. 7).

**Table 2. The dispersant packing density on nanoparticles measured by TGA and XPS respectively has been compared with that measured on flat surfaces.**

| dispersant | binding affinity | dispersant/nm² (nanoparticle) measured with TGA | dispersant/nm² (nanoparticle) measured with XPS | dispersant/nm² (flat) measured with XPS | EG/nm² (nano-particle) | EG/nm² (flat) |
|---|---|---|---|---|---|---|
| PEG(M_{W} 5 kDa)-nitroDOPA | high | 2.8±0.3 | 2.5±0.2 | 0.31±0.01 | 296±27 | 36±2 |
| PEG(M_{W} 5 kDa)-nitrodopamine | high | 2.6±0.2 | 2.5±0.4 | 0.26±0.01 | 290±40 | 30±1 |
| PEG(M_{W} 5 kDa)-mimosine | too high | 2.5±0.9 | 2.2±0.3 | 0.32±0.01 | 247±37 | 36±1 |
| PEG(M_{W} 5 kDa)-DOPA | intermediate | 2.4±0.9 | 1.8±0.4 | 0.26±0.00 | 210±50 | 30±0 |
| PEG(M_{W} 5 kDa)-dopamine | low | 2.3±0.5 | 2.2±0.2 | 0.22±0.01 | 254±25 | 25±1 |
| PEG(M_{W} 5 kDa) -hydroxy-dopamine | low | 1.7±0.6 | 2.2±0.2 | 0.15±0.00 | 254±26 | 17±0 |
| PEG(M_{W} 5 kDa)-hydroxy-pyridine | low | 1.8±0.6 | 1.7±0.1 | 0.22±0.01 | 197±16 | 25±1 |
| PEG(M_{W} 5 kDa)-hydroxy-pyrone | low | 1.8±0.2 | 2.1±0.1 | 0.11±0.00 | 244±13 | 13±0 |
| PEG(M_{W} 5 kDa)-COOH | low | 1.3±1.1 | 1.4±0.3 | 0.10±0.00 | 162±37 | 11±0 |

The obtained percentage weight losses measured between 200 and 400 °C by TGA (see Table 1) were converted into dispersant packing densities assuming spherical iron oxide nanoparticles with the core diameter distribution shown in Fig. 2 and an Fe₃O₄ density of 5.18 g/cm³ (Table 2). A clear trend towards higher particle stability with higher dispersant packing density was observed (see Table 2 and Fig. 3). Furthermore, if particles were heated to 90°C, before excessive dispersants were removed by filtration, the packing densities of PEG(M_{W} 5 kDa)-nitroDOPA and PEG(M_{W} 5 kDa)-mimosine stabilized nanoparticles remained in the range of packing densities of as-stabilized nanoparticles whereas that of PEG(M_{W} 5 kDa)-dopamine stabilized nanoparticles decreased to 26% of the initial packing density. This correlated well with the filtration experiments shown in Fig. 3b where the binding reversibility of dopamine on iron oxide surfaces was markedly higher than for mimosine and nitroDOPA.

The exothermic reaction around 255°C seen in differential scanning calorimetry (DSC) measurements for uncoated nanoparticles coincided with a mass loss of 20 wt% in TGA and corresponded to decomposition of weakly physisorbed impurities on the iron oxide nanoparticle surface (Fig. 7). A similar exothermic peak was measured for iron oxide nanoparticles stabilized with only 1 mg_{dispersant}/mg_{particle}. If particles were coated with 2 mg_{dispersant}/mg_{particle}, two exothermic reactions around 255 °C and 380 °C were observed. The first reaction was assigned to the decomposition of remaining impurities. The second exothermic reaction, which coincided with the main mass loss was determined as equal amounts of CO₂ and H₂O (measured in TGA coupled to a mass spectrometer) and was assigned to the decomposition of dispersants. For particles stabilized with at least 4 mg_{dispersant}/mg_{particle}, hardly any exothermic peak could be seen at 250°C whereas the exothermic decomposition of PEG around 410 °C was evident. These results indicate that nitroDOPA could replace physisorbed impurities if particles were stabilized with a sufficient amount of dispersants.

Dispersants adsorbed on flat Fe₃O₄ surfaces were analyzed by XPS. The atomic ratio of C_{PEG} to C_{aliphatic}, derived from the deconvoluted XPS C1s spectra, in combination with the atomic ratio of C_{PEG}:Fe can be taken as a measure of the binding affinity of the dispersants through the ability to replace aliphatic carbon contaminations. The atomic ratios of C_{PEG}:C_{aliphatic} and C_{PEG}:Fe increased with increasing nanoparticle stability (Fig. 8 and Fig. 9) and support DSC results where nitroDOPA was shown to replace weakly physisorbed carbon contaminations while low affinity anchor groups failed to do so. Moreover, high affinity dispersants resulted in higher dispersant packing densities compared to their low affinity counterparts (Table 2). Furthermore, a good linear correlation of the XPS atomic C_{PEG}:Fe ratios was obtained between catechol-based dispersants adsorbed on flat surfaces and on nanoparticles, respectively (Fig. 8b). This indicates that the binding affinity of anchoring groups is independent on surface curvature, in contrast to the packing density which is considerably higher on nanoparticles than on flat surfaces (Table 2).

The DSC, DLS, TGA and XPS results all showed that nitroDOPA and nitrodopamine were superior in terms of providing a high dispersant density on the nanoparticles, which directly correlated with irreversible binding and perfect particle stability under physiologic conditions.

### Example 3: Functionalization of iron oxide nanoparticles

The good control especially over the interfacial chemistry of iron oxide nanoparticles stabilized with said dispersants allowed further efficient and controlled surface functionalization. For that purpose, iron oxide nanoparticles were stabilized with a mixture of biotinylated PEG-nitroDOPA and unfunctioanlized PEG-nitroDOPA. Neutravidin was immobilized on SiO₂ coated quartz crystals through the formation of biotinylated supported lipid bilayers, mimicking a cell membrane. Fast binding of biotinylated iron oxide nanoparticles to neutravidin presenting surfaces could be measured with quartz crystal microbalance with dissipation monitoring (QCM-D). Specific binding of the biotin functionalized particles is demonstrated by the much higher adsorbed amount of these nanoparticles compared to unfunctionalized nanoparticles (Figure 10).

### Example 4: Investigation of binding mechanism

A deeper understanding of how these anchors bind opens up the possibility to predict binding affinities of different anchor groups towards desired oxides and to optimize their binding affinities, thus selecting a suitable anchor group for a specific application without laborious screening tests. Several investigations to gain knowledge on how the anchor groups bind have been performed such as FTIR, EPR, UV/VIS and NEXAFS. The results are outlined below.

*FTIR studies:* Interactions of nitroDOPA, nitrodopamine (in this Example referred to as nitrocatechols), DOPA, dopamine (in this Example referred to as catechols) and mimosine with iron, iron oxide and as a comparison TiO₂ were studied by complexing these anchors with Fe²⁺ and Fe³⁺ ions and adsorbing them on iron oxide and TiO₂ nanoparticles respectively. No differences in FTIR spectra of iron oxide and TiO₂ nanoparticles stabilized with the respective anchors were measured whether anchors were adsorbed from EtOH or DMF based solutions respectively. The FTIR spectra revealed two pronounced peaks, one at 1280 cm⁻¹ assigned to in-plane C-O stretching vibrations and one at 1496 cm⁻¹ assigned to tangential normal C-C vibration modes of the aromatic ring upon adsorption especially of nitrocatechols on iron oxide nanoparticles (Figure 11). Similar but considerably smaller peaks were also apparent for catechols and mimosine. The intensities of these peaks have been reported to be an indication of the extent of electron delocalization and thus binding strength of catechols with metal ions.

FTIR spectra of nitrocatechols adsorbed on iron oxide nanoparticles resembled more closely those of nitrocatechols complexed with Fe²⁺ compared to Fe³⁺. While the C-C ring vibration did not change for nitroDOPA whether complexed with iron ions or adsorbed on iron oxide nanoparticles compared to the pure control, the C-C ring vibration of nitrodopamine was slightly shifted towards higher wavenumbers upon adsorption on iron oxide nanoparticles and complexation with Fe³⁺ ions compared to pure and Fe²⁺ complexed nitrodopamine (Table 3).

**Table 3: FTIR peak locations. FITR peak wavenumbers of anchors complexed with iron ions and adsorbed on iron oxide and TiO₂ nanoparticles, respectively.**

| | nitroDOPA (cm⁻¹) | nitrodopamine (cm⁻¹) | DOPA (cm⁻¹) | dopamine (cm⁻¹) | mimosine (cm⁻¹) |
|---|---|---|---|---|---|
| C-O, reference | 1290 | 1294 | 1282 | 1286 | 1353 |
| C-O, Fe²⁺ | 1284 | 1278 | 1287 | 1286 | 1343 |
| C-O, Fe³⁺ | 1292 | 1290 | 1288 | 1286 | 1347 |
| C-O, iron oxide nanoparticle | 1281 | 1277 | 1263 | 1287 | 1350 |
| C-O, TiO₂ nanoparticle | 1286 | 1286 | 1269 | 1271 | 1350 |
| C-C, ring, reference | 1498 | 1493 | 1499 | 1499 | 1528 |
| C-C, ring, Fe²⁺ | 1497 | 1492 | 1493 | 1501 | 1520 |
| C-C, ring, Fe³⁺ | 1500 | 1497 | 1490 | 1501 | 1520 |
| C-C, ring, iron oxide nanoparticle | 1497 | 1495 | 1487 | 1487 | 1527 |
| C-C, ring, TiO₂ nanoparticle | 1500 | 1500 | 1481 | 1491 | 1531 |
| O-H, reference | 1341 | 1340 | 1299 | 1320 | 1377 |
| O-H, Fe²⁺ | | | | 1320 | |
| O-H, Fe³⁺ | | | | 1320 | |
| symmetric NO₂, reference | 1331 | 1321 | | | |
| symmetric NO₂, Fe²⁺ | 1331 | 1331 | | | |
| symmetric NO₂, Fe³⁺ | 1333 | 1332 | | | |
| symmetric NO₂, iron oxide nanoparticle | 1319 | 1317 | | | |
| symmetric NO₂, TiO₂ nanoparticle | 1319 | 1321 | | | |
| asymmetric NO₂, reference | 1535 | 1541 | | | |
| asymmetric NO₂, Fe²⁺ | 1534 | 1535 | | | |
| asymmetric NO₂, Fe³⁺ | 1537 | 1535 | | | |
| asymmetric NO₂, iron oxide nanoparticle | 1554 | 1554 | | | |
| asymmetric NO₂, TiO₂ nanoparticle | 1564 | 1562 | | | |

The asymmetric C-O ring vibration peak of nitrocatechols was shifted towards lower wavenumbers upon iron complexation and adsorption on iron oxide nanoparticles, respectively. Furthermore, especially the C-O ring vibration peak of Fe²⁺-complexed nitrocatechols was gradually shifted towards lower wavenumbers with time as could be seen by comparing spectra of complexes after 1 h and 24 h equilibration time at 50°C (Figure 12).

The peak around 1340 cm⁻¹ for nitrocatechols, assigned to in plane OH bending vibrations disappeared upon complexation with iron at 50°C for 24 h and adsorption on iron oxide nanoparticles while it remained unchanged if complexed for 1 h at RT with Fe²⁺ ions for nitroDOPA and with Fe²⁺ and Fe³⁺ ions for nitrodopamine.

Furthermore, the symmetric and asymmetric NO₂ vibration peaks were shifted markedly upon adsorption on iron oxide nanoparticles while no significant shift was observed for nitrocatechols complexed with Fe²⁺ and Fe³⁺.

Differences in FTIR spectra of DOPA complexed with Fe²⁺ and Fe³⁺ and that of DOPA adsorbed on iron oxide nanoparticles were more pronounced relative the same comparison for the nitrocatechols (Table 3). Furthermore, especially the C-C ring and C-O ring vibrations were considerably weaker for catechols compared to nitrocatechols if adsorbed on iron oxide nanoparticles indicating a lower surface coverage and less pronounced electron delocalization of DOPA compared to nitrocatechols. Additionally, these peaks became weaker for DOPA/iron complexes if kept at 50°C for 24 h compared to complexes kept at RT for 1 h which suggests slow, iron-catalyzed DOPA degradation. Whereas the C-C ring vibrations of nitrocatechols adsorbed on iron oxide nanoparticles were only slightly shifted towards higher wavenumbers, it was shifted considerably towards lower wavenumbers for catechols adsorbed on iron oxide nanoparticles compared to reference catechols.

The similarities of FTIR spectra of mimosine complexed with Fe²⁺ and Fe³⁺ ions and that of mimosine adsorbed on iron oxide nanoparticles were striking. Furthermore, in contrast to catechol and nitrocatechol/iron complexes, FTIR spectra of mimosine/iron complexes did not change significantly with complexation time and temperature. This indicates a fast reaction between mimosine and iron which results in stable complexes. Similar to nitrocatechols but in contrast to catechols, adsorption of mimosine on iron oxide nanoparticles and complexation with iron ions resulted in a shift of the C-C ring vibration peak towards higher wavenumbers for mimosine. However, in contrast to catechols and nitrocatechols, the C-O ring vibration of mimosine adsorbed on iron oxide nanoparticles was shifted towards higher wavenumbers upon adsorption on iron oxide nanoparticles compared to pure compounds, which suggests strengthening of the C-O bond upon complexation to iron. This indicates a very strong and stable bond of mimosine to iron ions. The even stronger bond indicated for mimosine than for nitrocatechols provides a possible explanation to the observed dissolution of iron oxide nanoparticles stabilized by mimosine dispersants relative those stabilized by nitrocatechol dispersants. A too high affinity will lead to dissolution by extraction of the metal ions out of a inorganic non-metallic nanoparticle and thus overall poor particle stability.

A comparison of FTIR spectra of anchor groups adsorbed on iron oxide and TiO₂ nanoparticles reveals significant differences. Relative peak intensities of the C-C and C-O peaks of anchors to that of oxides are considerably higher if nitrocatechols are adsorbed on iron oxide compared to TiO₂ nanoparticles. Furthermore, asymmetric and symmetric NO₂ vibrations were more pronounced if nitrocatechols were adsorbed on iron oxide compared to TiO₂ nanoparticles. However, peak positions were only at slightly higher wavenumbers for anchors adsorbed on TiO₂ nanoparticles compared to those adsorbed on iron oxide nanoparticles.

*EPR studies:* Electron paramagnetic resonance (EPR) spectra of iron oxide nanoparticles stabilized with poly(ethylene glycol)-nitroDOPA where the poly(ethylene-glycol) had a molecular weight of 5 kDa (PEG(M_{W} 5 kDa)-nitroDOPA), PEG(M_{W} 5 kDa)-mimosine and PEG(M_{W} 5 kDa)-dopamine clearly revealed the presence of radicals (g=2.06) upon adsorption of the respective dispersants (Figure 13a). No radicals were seen for uncoated iron oxide and TiO₂ nanoparticles or for pure dispersants demonstrating that electron delocalization was involved in the binding of dispersants to iron oxide nanoparticles. Similar to FTIR studies, no difference in EPR spectra was measured whether dispersants were adsorbed onto iron oxide and TiO₂ nanoparticles from EtOH or DMF based solutions respectively. In addition to the radical signal, a signal around g=4.18 seen for PEG(M_{W} 5 kDa)-nitroDOPA, PEG(M_{W} 5 kDa)-dopamine and PEG(M_{W} 5 kDa)-mimosine stabilized iron oxide nanoparticles, assigned to octahedrally distorted, magnetically decoupled Fe³⁺ , appeared upon dispersant adsorption (Figure 13b). No significant Fe³⁺ signal was detected for uncoated nanoparticles. Furthermore, radicals and magnetically decoupled Fe³⁺ signals of stabilized iron oxide nanoparticles disappeared upon heating of iron oxide nanoparticles for 2 h at 200 °C showing that these two EPR signals were related (Figure 13c). The relative peak-to-peak intensities of radicals:Fe³⁺ EPR signals were with 43 ± 2 highest for PEG(M_{W} 5 kDa)-nitroDOPA stabilized nanoparticles, followed by PEG(M_{W} 5 kDa)-mimosine with 37 ± 0 and PEG(M_{W} 5 kDa)-dopamine with 29 ± 2. Thus, the density of delocalized electrons finally binding to Fe³⁺ is considerably higher for nitroDOPA compared to dopamine.

Moreover, the line width ΔB of the superparamagnetic EPR peak around g=2.06 of PEG(M_{W} 5 kDa)-anchor stabilized nanoparticles was considerably lower compared to that of unstabilized iron oxide nanoparticles which still had precursors and impurities on their surface, indicating less magnetic interactions of stabilized compared to unstabilized nanoparticles. The stabilization of individual iron oxide cores with dispersants prevents magnetic interactions between iron oxide nanoparticle cores due to larger interparticle distances caused by the stabilizing dispersant layer which results in a narrower superparamagnetic peaks compared to magnetically interacting iron oxide cores. Interestingly, electron delocalization was also observed for PEG(M_{W} 5 kDa)-nitroDOPA and PEG(M_{W} 5 kDa)-dopamine stabilized TiO₂ nanoparticles in contrast to TiO₂ nanoparticles stabilized with PEG(M_{W} 5 kDa)-mimosine (Figure 13c). However, no radicals (delocalized electrons) were measured for pure dispersants (Figure 13d). The different degree of electron delocalization of the different anchor groups adsorbed on TiO₂ nanoparticles was visible in color differences of TiO₂ nanoparticle dispersions stabilized with different dispersants (Figure 13f).

*UV*/*VIS spectroscopy*: Five different anchor groups have been complexed with Fe²⁺ and Fe³⁺ ions at a molar ratio of 1:1 in Millipore water, Tris(hydroxymethyl)aminomethane containing 150 mM NaCl (Tris) buffer, DMF and EtOH respectively. The pH of complex solutions in Millipore water varied but was always slightly acidic due to the addition of iron chlorides and Tris was chosen to buffer the solutions to pH=7.4 because it has been reported not to interfere with iron ions.

Whereas peak locations and intensities of UV/VIS spectra of reference nitrocatechols closely resembled each other if aliquotted in Millipore water and Tris respectively, only peak locations were identical between these two anchors if complexed with iron ions in DMF while relative peak intensities differed markedly. Nitrocatechols are deprotonated in Tris whereas they are protonated in Millipore water. The close resemblance of UV/VIS spectra of nitrocatechols dissolved in EtOH and Millipore water indicates that nitrocatechols are also protonated in ethanol whereas they are deprotonated in DMF as indicated by the similarities of UV/VIS spectra of reference nitrocatechols dispersed in Tris and DMF, respectively.

Only minor changes in UV/VIS spectra of catechols complexed with Fe²⁺ ions in Millipore water and EtOH were observed compared to uncomplexed reference spectra (Table 4, Figures 14-16). However, changes of UV/VIS spectra of catechols complexed with Fe²⁺ in DMF and Tris were larger.

**Table 4: UV/VIS peak locations. UV/VIS peak locations of anchors complexed with Fe²⁺ and Fe³⁺ ions in Millipore water and Tris buffer respectively.**

| | nitroDOPA | nitrodopamine | DOPA | dopamine | mimosine |
|---|---|---|---|---|---|
| π→π*, control, MQ | 246 | 245 | 279 | 279 | 278 |
| π→π* transition, Fe²⁺, MQ | 246 | 245 | 279 | 279 | 287 |
| π→π* transition, Fe³⁺, MQ | 260 | 259 | 281 | 282 | 284 |
| π→π*,control, Tris | | 286 | 279 | 279 | 280 |
| π→π* transition, Fe²⁺, Tris | 267 | 268 | 292 | 291 | 288 |
| π→π* transition, Fe³⁺, Tris | 266 | 264 | 287 | 286 | 282 |
| π→π*, control, EtOH | 312 | 308 | 281 | 282 | |
| π→π* transition, Fe²⁺, EtOH | 315 | 308 | | 284 | |
| π→π* transition, Fe³⁺, EtOH | 321 | 308 | 330 | 281 | 243 |
| π→π* transition, Fe²⁺, DMF | | | 292 | 285 | 291 |
| π→π* transition, Fe³⁺, DMF | | | | 285 | |
| HOMO→ LUMO, control, MQ | 352 | 352 | | | |
| HOMO→ LUMO, Fe²⁺, MQ | 353 | 352 | | | |
| HOMO→ LUMO, Fe³⁺, MQ | 318 | 315 | | | |
| HOMO→ LUMO, control, Tris | 422 | 422 | | | |
| HOMO→ LUMO, Fe²⁺, Tris | 414 | 418 | | | |
| HOMO→ LUMO, Fe³⁺, Tris | 327 | 329 | | | |
| HOMO→ LUMO, control, EtOH | 355 | 357 | | | |
| HOMO→ LUMO, Fe²⁺, EtOH | 381 | 353 | 322 | | 320 |
| HOMO→ LUMO, Fe³⁺, EtOH | 372 | 351 | 357 | 332, 357 | 367 |
| HOMO→ LUMO, control, DMF | 441 | 446 | | | |
| HOMO→ LUMO, Fe²⁺, DMF | 407 | 421 | | | |
| HOMO→ LUMO, Fe³⁺, DMF | 313, 365 | 363 | | | |
| HOMO-1→ LUMO, control, DMF | 365 | 362 | | | |
| HOMO-1→ LUMO, Fe³⁺, MQ | 391 | | | | |
| HOMO-1→ LUMO, Fe³⁺, Tris | 406 | 390 | | | |
| HOMO-1→ LUMO, Fe²⁺, DMF | 368 | 405 | | | |
| HOMO-1→ LUMO, Fe3+, DMF | 313 | 350 | | | |
| charge transfer, Fe^{2+,} MQ | | 347 | | | |
| charge transfer, Fe³⁺, MQ | 391 | | | | 464 |
| charge transfer, Fe²⁺, Tris | 414 | 390 | 403, 749 | 405, 744 | 513 |
| charge transfer, Fe³⁺, Tris | 406 | 418 | 575 | 576 | 452 |
| charge transfer, Fe²⁺, EtOH | | 422 | | | |
| charge transfer, Fe³⁺, EtOH | 670 | | | | |
| charge transfer, Fe²⁺, DMF | | 405 | 575 | 565 | 447 |
| charge transfer, Fe³⁺, DMF | 653 | 602 | 317 | 360,602 | 318 |

A broad adsorption bands between 500 and 700 nm appeared upon complexation of nitrocatechols with Fe²⁺ while a sharper electron transfer peak became evident upon complexation of mimosine with Fe^{2+.}

The absorption peak originating from π → π* ransitions of benzene remained unchanged for catechols and only slightly shifted towards higher wavelengths for mimosine upon complexation with Fe³⁺. However, it was shifted considerably if nitrocatechols were complexed with Fe³⁺ (Figure 16 and Table 4).

The peak around 350 nm for pure nitrocatechols dispersed in Millipore water and EtOH and between 420 and 440 nm for nitrocatechols dispersed in Tris and DMF respectively was assigned to HOMO→LUMO transitions resulting from a charge transfer from the HOMO localized on the benzene ring, which has π character to the π* UMO mainly localized on the nitro group. A similar electron transfer from the catechol to the nitro group results in the HOMO-1→LUMO π→π* transition, which was observed at 365 nm for uncomplexed, in DMF dispersed and 313 nm for Fe³⁺ complexed nitrocatechols. Furthermore, anchor-iron electron transfer reactions were seen between 400 and 800 nm for Fe³⁺ complexed with catechols and nitrocatechols, respectively, whereas mimosine/Fe³⁺ complexes resulted in sharper charge transfer peaks where the peak location depended on the solvent, these complexes were formed in. This charge transfer peak was also seen in UV/VIS spectra of supernatants of iron oxide nanoparticles stabilized with PEG(M_{W} 5 kDa)-mimosine but not in supernatants of iron oxide nanoparticles stabilized with the other investigated dispersants (Fig. 17) and demonstrates a slow dissolution of iron oxide nanoparticles by mimosine through mimosine/Fe³⁺ complex formation, as discussed earlier.

Furthermore, π → π * ring absorption of catechols and mimosine complexed with iron ions was shifted towards higher wavelengths compared to uncomplexed compounds, hardly any change in the position of the π → π * ring peak could be detected for nitrocatechols complexed with iron ions (Table 4). However, this peak became considerably more pronounced upon complexation of nitrocatechols with iron ions.

The HOMO→LUMO catechol-NO₂ electron transfer peak measured for nitrocatechols was broadened markedly upon complexation in Tris. Furthermore, the shift towards lower wavelengths was more pronounced if nitrocatechols were complexed with Fe³⁺ compared to Fe²⁺ ions. Moreover, while ligand-charge electron transfer reaction resulted in clear peaks for mimosine and catechol iron complexes in Millipore water disappeared after 8 days if kept at RT and 1 day if kept at 50°C (see Fig. 20). However, π → π * ring absorption peaks of nitrocatechol-iron complexes were still visible after 8 days kept at RT or 1 day at 50°C. Furthermore, the π → π * ring absorption peak position remained unchanged for nitrocatechol/Fe³⁺ complexes whereas they were shifted towards lower energies for nitrocatechol/Fe²⁺ complexes. Additionally, the considerable broadening of the HOMO→LUMO transition of nitrocatechol/iron complexes was observed almost instantaneously but remained constant for at least 8 d at room temperature or 1 d at 50°C.

While UV/VIS spectra of complexed mimosine/Fe³⁺ were independent of the complexation time and temperature indicating high stability of these complexes, broadening of the charge-transfer peak was seen for mimosine/Fe²⁺ complexes especially if kept at 50°C for 24 h.

*NEXAFS studies*: The decrease in intensity of the peak at 527 eV and the appearance of the peak at 529 nm in the O K-edge near edge X-ray absorption fine structure (NEXAFS) spectra of mimosine adsorbed on flat iron oxide surfaces is what would be expected for a change in the iron oxide stoichiometry from Fe₃O₄ to Fe₂O₃ (Figure 18a). This stands in contrast to DOPA and dopamine where the O-edge spectra resembled that of uncoated iron oxide. Furthermore, the shape of the peak around 715 eV of the Fe L-edge NEXAFS spectra might indicate that spins were oriented perpendicular to the surface if mimosine was adsorbed on iron oxide surfaces in contrast to DOPA and dopamine (Figure 18b). This change in surface chemistry and potential crystallographic rearrangement upon binding of mimosine to iron oxide, which was not observed for DOPA or dopamine correlates with the stronger interaction of mimosine than for the catechols observed by FTIR, EPR and UV/VIS.

*Discussion on binding mechanism:* Electron transfer between catechols and TiO₂ surfaces has been described in detail in literature. Therefore, radicals which resulted in an EPR signal at g=2.06 can be assigned to delocalized electrons resulting from strong interactions of anchors with the respective oxide. The absence of radicals upon adsorption of mimosine on TiO₂ nanoparticles shows a weaker interaction and thus binding strength of mimosine to TiO₂ compared to nitroDOPA and dopamine. However, delocalized electrons were measured for all investigated anchors if adsorbed on iron oxide nanoparticles. The considerably higher ratio of delocalized electrons to magnetically decoupled Fe³⁺ ions measured with EPR spectroscopy for PEG(M_{w} 5 kDa)-nitroDOPA adsorbed on iron oxide nanoparticles compared to PEG(M_{w} 5 kDa)-dopamine matches the higher nanoparticle stability of PEG(M_{w} 5 kDa)-nitroDOPA compared to PEG(M_{w} 5 kDa)-dopamine stabilized iron oxide nanoparticles especially at elevated temperatures as was shown in an earlier empirical study.

Furthermore, the simultaneous appearance and after dispersant decomposition disappearance of EPR signals of radicals and octahedrally distorted Fe³⁺ ions upon adsorption of dispersants on iron oxide nanoparticles evidenced that these two signals are related to each other. Therefore, the octahedrally distorted Fe³⁺ signal results from surface Fe³⁺ ions to which anchors have bound. The higher density of delocalized electrons of nitrocatechols interacting with Fe³⁺ ions compared to catechol/ Fe³⁺ complexes, which is associated with a more covalent rather than ionic bond between these anchors and iron ions thus correlates with and presents an explanation to the difference in binding affinity and hence also iron oxide nanoparticle stability previously observed. The decreasing intensities of the C-C stretching and C-O ring vibrations from nitrocatechols to mimosine and catechols if adsorbed on iron oxide nanoparticles measured with FTIR spectroscopy further corroborated differences in the amount of delocalized electrons involved in the binding of these anchors to iron oxide and TiO₂ surfaces.

The anchors investigated here have been shown to bind to TiO₂ in a deprotonated form. This is also true for the adsorption of anchors on iron oxide as was shown by FTIR analysis where the OH peaks seen for reference nitrocatechol spectra disappeared for nitrocatechols adsorbed on iron oxide nanopartilces or complexed with iron ions respectively. The pKₐ value of one of the hydroxyl-group of nitrocatechols which is around 6.7 whereas pKₐ values of hydroxyl-groups of catechols are above 9. Thus, the partial deprotonation of nitrocatechols leads to a facilitated complexation of iron ions in a pH range between 6.5 and 9 compared to catechols. Similar to reference UV/VIS spectra of nitrocatechols dissolved in Tris, DMF based nitrocatechol reference spectra showed pronounced HOMO→LUMO transitions indicating enhanced electron transfer between the benzyl ring and the nitro group compared to Millipore water and EtOH based nitrocatechol reference spectra. This indicates that HOMO→LUMO transitions are facilitated if nitrocatechols are deprotonated. It further implies that nitrocatechols are not only deprotonated if dissolved in Tris, where the pH>pKₐ, but also if dispersed in DMF, in contrast to nitrocatechols dispersed in Millipore water, where the pH<pKₐ and in EtOH.

Furthermore, the electronic structure of the nitro group changed significantly upon complexation of nitrocatechols with iron. This was indicated by a considerable broadening of the HOMO →LUMO transition of nitrocatechol/iron complexes if dispersed in Tris, DMF and EtOH, respectively. However, broadening of the HOMO →LUMO transition of nitrocatechols dispersed in Millipore water upon complexation with Fe³⁺ was small and even negligible upon complexation with Fe²⁺. Furthermore, no significant shifts in the asymmetric and symmetric NO₂ vibrations were measured in FTIR studies for nitrocatechols upon complexation with iron ions in Millipore water. These differences between Millipore water and Tris based nitrocatechol/iron complexes further corroborate the finding that changes in the electron density on the nitro group upon complexation of iron ions is greatly facilitated if nitrocatechols are deprotonated.

According to UV/VIS spectra, nitrocatechols strongly bind to Fe²⁺ and Fe³⁺ions if dispersed in DMF and EtOH, as they were during the adsorption of dispersants on iron oxide nanoparticles. Freshly synthesized Fe₃O₄ nanoparticles present Fe²⁺ and Fe³⁺ on their surface. FTIR spectra of nitrocatechols adsorbed on iron oxide nanoparticles resembled more closely FTIR spectra of nitrocatechols complexed with Fe²⁺ than Fe³⁺ if incubated at 50°C for 24 h. However, the peak at g=2.18 observed in EPR spectra indicates that nitrocatechols bind to Fe³⁺ rather than Fe²⁺. This apparent discrepancy might be explained by a redox reaction between nitrocatechols and iron ions. According to FTIR spectra, nitrocatechols initially bind to Fe²⁺. They then might oxidize Fe²⁺ to Fe³⁺ while being reduced (Figure 12). Furthermore, a redox reaction between nitrocatechols and iron ions could explain the increased electron density on the NO₂ leading to the marked changes in the symmetric and asymmetric NO₂ vibrations measured with FTIR.

The markedly lower intensity of the symmetric and asymmetric NO₂ peak of nitrocatechols adsorbed on TiO₂ nanoparticles compared to iron oxide nanoparticles hints to significant differences in the electronic structure of nitrocatechols adsorbed on iron oxide and those adsorbed on TiO₂. This supports the interpretation of an increased electron density at the nitro group resulting from a redox reaction between Fe²⁺ and nitrocatechols which occurs if nitrocatechols are adsorbed on iron oxide nanoparticles but not if adsorbed on TiO₂ nanoparticles.

The absence of an electronegative NO₂ group of catechols compared to nitrocatechols not only leads to pKₐ values of catechol OH groups >9 but also renders catechols more prone to oxidation compared to nitrocatechols. Furthermore, the additional carboxy group increases the pKₐ values of the hydroxyl groups of DOPA compared to dopamine and renders the former anchors less prone to oxidation. The higher intensities of C-C and C-O vibrations seen on FTIR spectra for DOPA adsorbed on iron oxide nanoparticles compared to dopamine indicate a higher affinity of DOPA towards iron compared to dopamine. The shifts of the C-C ring vibration peaks of DOPA complexed with iron ions or catechols adsorbed on iron oxide nanoparticles compared to the uncomplexed reference spectra is in contrast to nitrocatechols, where no significant change in the position of the C-C ring vibration peak was seen upon complexation with iron ions or adsorption on iron oxide nanoparticles. This points to a change in the electronic structure of the catechol ring whereas the aromatic structure of nitrocatechols seems to be retained even after adsorption on iron oxide nanoparticles. These shifts were attributed to iron catalyzed catechol degradation which can result in carboxy containing species (Figure 19). Carboxy groups have been shown in example 1 to poorly bind to iron oxide nanoparticles which could explain the lower packing density of catechols compared to nitrocatechols seen in FTIR spectra and the lower degree of delocalization measured for PEG(M_{w} 5 kDa)-dopamine adsorbed on iron oxide nanoparticles compared to PEG(M_{w} 5 kDa)-nitroDOPA in EPR spectra. Iron catalyzed catechol degradation was further supported by the marked decrease in the π→π* and electron transfer peaks in UV/VIS spectra of catechols complexed with iron in Millipore water if kept for 8 d at RT or 1 d at 50°C.

No time dependent changes in FTIR spectra of mimosine/iron complexes were observed. Furthermore, the UV/VIS π→π* transition peak of Millipore water based complex solutions did not change with time and only minor time dependent changes of UV/VIS electron transition peak were measured. This indicates a high stability of mimosine/iron complexes and might partially be due to the low pKₐ values of mimosine. Thus, mimosine is deprotonated irrespective whether dispersed in Millipore water, Tris, DMF or EtOH and can therefore easily complex iron ions. The close resemblance of FTIR peak locations of mimosine adsorbed on iron oxide and TiO₂ nanoparticles respectively hints to a close similarity of the electronic structure of mimosine irrespective whether adsorbed on iron oxide or TiO₂ nanoparticles. This indicates that no redox reaction of mimosine with iron ions occur upon adsorption of mimosine on iron oxide nanoparticles (Figure 11). However, the relative FTIR peak intensities of mimosine adsorbed on TiO₂ nanoparticles was considerably lower compared to those where mimosine had been adsorbed on iron oxide nanoparticles. This indicates a higher affinity of mimosine towards iron oxide compared to TiO₂ and is supported by EPR spectra, where delocalized electrons were clearly detected if PEG(M_{w} 5 kDa)-mimosine was adsorbed on iron oxide nanoparticles whereas no delocalized electrons were seen upon adsorption of PEG(M_{w} 5 kDa)-mimosine on TiO₂ nanoparticles and might hint to the fact, that mainly lonely electron pairs located at the deprotonated pyridine alcohol groups of mimosine are involved in the strong binding of mimosine to iron oxide but not to TiO₂.

The close similarity of the UV/VIS spectra of the supernatant of PEG(M_{w} 5 kDa)-mimosine stabilized iron oxide nanoparticles dispersed in Millipore water with that of Millipore based mimosine/Fe³⁺ complexes indicates that mimosine adsorbed on iron oxide nanoparticles binds to Fe³⁺ rather than Fe²⁺. This strong complexation of PEG(M_{w} 5 kDa)-mimosine might permanently remove Fe³⁺ ions and thus leads to gradual iron oxide nanoparticle dissolution. This is supported by the presence of PEG(MW 5 kDa)-mimosine/Fe³⁺ complexes in the supernatant of PEG(MW 5 kDa)-mimosine stabilized iron oxide nanoparticles as was found with UV/VIS spectroscopy. No such PEG(M_{w} 5 kDa)-anchor/iron ions complexes were found in the supernatant of the nitrocatechols and catechols (Figure 17).

*Conclusions on binding mechanism and prediction ofgood anchors:* The striking difference in binding affinities of these different anchor groups might be related to different pKₐ values. The nitro group lowers the pKₐ value of one of the hydroxyl groups leading to facilitated deprotonation which enhances the binding affinities of nitrocatechols towards iron oxide compared to catechols. While nitroDOPA and nitrodopamine, the two anchor groups which lead to highest particle stability, have one of their pKₐ values around 6.5, close to the point of zero charge (PZC) of iron oxide nanoparticles (6.7), DOPA, dopamine and hydroxydopamine have pKₐ >9 (Fig. 21). The binding affinity of catechols and catechol derivatives towards TiO₂ has been shown to scale with their Brønstead acidity [7] which is influenced by the carboxy group and might be one of the reasons for the higher affinity of DOPA compared to dopamine towards iron oxide. Furthermore, the aromatic nitrogen of mimosine and hydroxypyridine enhances the Brønstead acidity of these anchors compared to DOPA and dopamine, respectively, which might result in higher packing densities of dispersants containing pyridine-based anchor groups compared to their catechol-based counterparts. The electronegative nitro-group of nitrocatechols facilitates electron delocalization of these anchors. However, if dispersant anchors to create adlayers, such as mimosine, complex iron ions too strongly, iron oxide nanoparticles are gradually dissolved by these anchors. Therefore, there is an optimum binding affinity of anchors where they strongly bind dispersants to iron oxide nanoparticle surfaces while the complexation strength of these anchors is too low to degrade these nanoparticles. It can also be noted that a mere increase in the number of hydroxyl groups does not significantly increase the affinity of the dispersant, suggesting that binding occurs with the ring oriented orthogonal to the substrate, sterically allowing only two hydroxyl groups to form simultaneous bonds with the iron oxide surface.

Because of the close relationship between the binding affinity of these anchors towards cations and the respective oxide surface, knowledge gained on complexation studies can be translated into binding affinities to oxide surfaces. This renders easily acquirable UV/VIS spectra of anchors and anchor/cation complexes a powerful tool to estimate the extent of electrons which are delocalized upon complexation of anchors with the respective cations. Therefore, a first selection of potential anchors for oxide nanoparticle stabilization can be based on complexation constants and UV/VIS spectra. Especially the relative intensities of C-C ring and C-O stretch vibrations in FTIR spectra of anchors adsorbed on the respective oxide nanoparticles reveal further valuable information on the suitability of the chosen molecule as anchor for nanoparticle stabilization without the need to covalently couple spacer groups to these anchors. Well chosen anchor groups which have an optimal binding strength towards the respective oxide can then be covalently coupled to a suited spacer. The resulting dispersants allow to individually stabilize these oxide nanoparticles and to closely control their hydrodynamic diameter and surface functionalities. Finally, simple filtration/redispersion experiments and temperature dependent DLS measurements of so-stabilized inorganic non-metallic nanoparticles are a powerful way to verify good nanoparticle stability, in particular to quickly verify the irreversibility of a particular anchor and its propensity to dissolve nanoparticles by comparing particle size distribution and scattering count rate.

*Discussion on particle dispersion stability:* The investigated anchors were as described above used to stabilize nanoparticles, as a particular example iron oxide nanoparticles, for applications under physiological conditions using PEG(M_{w} 5 kDa) spacers. The hydrodynamic diameter of iron oxide nanoparticles stabilized with PEG(M_{w} 5 kDa)-nitroDOPA, PEG(M_{w} 5 kDa)-nitrodopamine, PEG(M_{w} 5 kDa)-mimosine, PEG(M_{w} 5 kDa)-DOPA and PEG(M_{w} 5 kDa)-hydroxypyridine dispersed in HEPES at 40°C was considerably lower (23 nm) and the particle size distribution was narrower than for previously reported PEG(6)-dopamine stabilized iron oxide nanoparticles dispersed in phosphate buffered saline (PBS) at 37°C (≈90 nm) [3] despite similar iron oxide core diameters. An average dispersant shell thickness of 8.5 nm was calculated given an iron oxide core diameter of 6 nm and the hydrodynamic diameter of 23 nm. Superior particle stability and fewer agglomerates of PEG(M_{w} 5 kDa)-nitroDOPA and PEG(M_{w} 5 kDa)-nitrodopamine compared to PEG(M_{w} 5 kDa)-dopamine stabilized nanoparticles was also directly shown by the experiments. The lower stability of iron oxide nanoparticles coated with PEG(M_{w} 5 kDa)-dopamine compared to e.g. PEG(M_{w} 5 kDa)-nitrocatechol stabilized nanoparticles might partially be because dopamine is prone to oxidation especially in the presence of iron if kept aliquotted at room temperature, in contrast to e.g. nitrocatechols. However, the oxidation cannot be the sole reason, which was further supported by the different binding mechanisms described above.

For weak and intermediate affinity dispersants a solution temperature slightly below the corresponding PEG cloud point induced irreversible nanoparticle agglomeration. However, PEG(M_{w} 5 kDa)-nitroDOPA and PEG(M_{w} 5 kDa)-nitrodopamine stabilized iron oxide nanoparticles remained dispersed after repeated heating to 90°C. A plausible explanation for temperature induced irreversible agglomeration of nanoparticles stabilized with weak or intermediate affinity dispersants is that these dispersants dissociate faster at elevated temperatures.

The high binding affinity of some of the dispersants correlated strongly with the unsurpassed dispersant packing density shown herein. The maximum PEG(M_{w} 5 kDa)-catechol packing density previously reported was 0.3 PEG(M_{w} 5 kDa)/nm² obtained for triple anchor PEG(M_{w} 5 kDa)-(DOPA)₃ adsorbed on flat TiO₂ surfaces [8]. This density is comparable to that measured here for PEG(M_{w} 5 kDa)-nitroDOPA and PEG(M_{w} 5 kDa)-mimosine adsorbed on flat iron oxide surfaces (Table 2). Furthermore, the packing density of PEG-silanes adsorbed on Fe₃O₄ nanoparticles had been reported to be between 0.3 and 0.8 molecules/nm² for PEG molecular weights between 0.1 and 0.35 kDa [9]. Moreover, the maximum PEG(M_{w} 5 kDa) packing density measured on nanoparticles was 1.3 dispersants/nm² for PEG(M_{w} 5 kDa)-hydroxydopamine [1] adsorbed on iron oxide nanoparticles which is two times lower than the packing density measured for the nitrocatechol dispersants described in this invention.

The extremely high dispersant packing density described here appears, at first glance, to be too high for a grafting-to immobilization process that requires diffusion of large polymers through a brush of already adsorbed polymers to reach completion. However, account must be taken of the high curvature of the nanoparticles. This is in agreement with the thiol packing density which has been reported to be up to 2.2 times higher for PEG-SH and 23 times for polystyrene-thiols (PS-SH) if adsorbed on Au nanoparticles compared to 2D Au surfaces. The conical volume that can be occupied by each immobilized PEG chain will significantly reduce steric inter-chain repulsion, leading to a different chain density profile and thus different dispersant packing densities on highly curved surfaces of nanoparticles. However, good correlation of the XPS C_{PEG}:Fe atomic ratios between dispersants adsorbed on flat, 2D surfaces and on 3D nanoparticles (Fig. 8b) still allows for screening of dispersants on 2D surfaces where more surface sensitive and quantitative characterization techniques are at hand.

Irreversible binding of low molecular weight dispersants which withstand physiologic conditions allow for controlled and efficient surface functionalization of individual oxide nanoparticles, e.g. with antibodies for targeting purposes or additional labels such as fluorophores without the risk of ligand dissociation caused by weak dispersant-nanoparticle interactions. Thus, so-functionalized nanoparticles are promising candidates for *in vivo* applications. Furthermore, the hydrodynamic diameter of individually stabilized nanoparticles solely depends on the iron oxide core diameter and the dispersant layer thickness and can therefore easily be controlled by adjusting the dispersant molecular weight and iron oxide core diameter. This is of great relevance for passive targeting of e.g. tumor tissue, by the enhanced permeation and retention effect as well as, in concert with a more stable presentation of bioactive ligands, for the active and specific targeting of cellular and tissue components.

Furthermore, these particles can be freeze-dried and re-dispersed at the desired concentration and physiologic conditions, which facilitates particle handling considerably. Finally, catechols are known to have high affinity also towards a broad range of oxides such as but not limited to TiO₂ and ZnO. Therefore, these dispersants are likely to be applicable to individually stabilize oxide nanoparticles in general through the easy and cost-effective grafting-to method. Thus, these catechol-based dispersants pave the way for a variety of new applications of oxide nanoparticles where irreversible dispersant binding, good nanoparticle stability under harsh conditions, long shelf life, tailoring of the hydrodynamic diameter and interfacial chemistry, and easy particle handling are stringent requirements.

By the present invention individually stabilized sub-30 nm iron oxide nanoparticles wherein the dispersant packing density was twice as high as the currently highest reported PEG packing density were provided. The particle stability could be shown to be strongly correlated with the dispersant packing density, which in turn correlated with irreversible anchor affinity for the oxide and no dissolution of the particle core. The reported iron oxide nanoparticle stability vastly exceeded that of previously reported similar particles, especially at medically relevant conditions of elevated temperature and ionic strength. Irreversible binding of high affinity anchor groups such as nitroDOPA and nitrodopamine allowed diluting and heating iron oxide nanoparticle suspensions without nanoparticle agglomeration. Inventive high affinity anchor groups therefore allow for quantitative tailoring of molar ratios of differently functionalized dispersants at the nanoparticle surface, resulting in e.g. targeted and multi-functional SPIONs for *in vivo* use.

### Chemical characterization of the materials

Mass spectroscopy analysis of the dispersants is shown in Fig. 22, wherein:
a) PEG(M_{w} 5 kDa)-nitroDOPA, b) PEG(M_{w} 5 kDa)-nitrodopamine, c) PEG(M_{w} 5 kDa)-mimosine, d) PEG(M_{w} 5 kDa)-DOPA, e) PEG(M_{w} 5 kDa)-dopamine, f) PEG(M_{w} 5 kDa)-hydroxypyridine, g) PEG(MW 5 kDa)-hydroxydopamine, h) PEG(M_{w} 5 kDa)-hydroxypyrone, i) PEG(M_{w} 5 kDa)-COOH. m/z values of peaks indicated with a star are reported in Table 5. They are in agreement with calculated masses of the respective protonated dispersants. Δm/z (x) is 44 Da, corresponding to the ethylene glycol repeat unit.

**Table 5. MALDI TOF analysis of dispersants.**

| | m/z * |
|---|---|
| PEG(M_{w} 5 kDa)-nitroDOPA | 4979 |
| PEG(M_{w} 5 kDa)-nitrodopamine | 5023 |
| PEG(M_{w} 5 kDa)-mimosine | 4848 |
| PEG(M_{w} 5 kDa)-DOPA | 5022 |
| PEG(M_{w} 5 kDa)-dopamine | 5135 |
| PEG(M_{w} 5 kDa)-hydroxydopamine | 5073 |
| PEG(M_{w} 5 kDa)-hydroxypyridine | 5067 |
| PEG(M_{w} 5 kDa)-hydroxypyrone | 5022 |
| PEG(M_{w} 5 kDa)-COOH | 5375 |

**Table 6. Microelement analysis of dispersants.**

| | C measured (at%) | C calculated (at%) | O measured (at%) | O calculated (at%) | N measured (at%) | N calculated (at%) |
|---|---|---|---|---|---|---|
| PEG(M_{w} 5 kDa)-nitroDOPA | 53.9 | 53.9 | 36.6 | 36.7 | 0.4 | 0.5 |
| PEG(M_{w}5kDa)-nitrodopamine | 54.2 | 54.1 | 36.3 | 36.4 | 0.3 | 0.5 |
| PEG(M_{w} 5 kDa)-DOPA | 53.6 | 54.5 | 36.5 | 36.3 | 0.2 | 0.3 |
| PEG(M_{w} 5 kDa)-dopamine | 54.5 | 54.7 | 36.0 | 36.0 | 0.2 | 0.3 |
| PEG(M_{w} 5 kDa)-hydroxydopamine | 54.0 | 54.4 | 36.4 | 36.3 | 0.5 | 0.3 |
| PEG(M_{w} 5 kDa)-mimosine | 54.1 | 54.2 | 36.4 | 36.3 | 0.4 | 0.5 |
| PEG(M_{w} 5 kDa)-hydroxypyridine | 53.5 | 54.6 | 36.0 | 35.9 | 0.4 | 0.5 |
| PEG(M_{w} 5 kDa)-Oring | 54.3 | 54.4 | 36.3 | 36.4 | 0.2 | 0.3 |
| PEG(M_{w} 5 kDa)-COOH | 54.4 | 54.3 | 36.8 | 36.6 | 0.0 | 0.0 |

### NMR analysis of dispersants

### PEG(M_{w} 5 kDa)-nitroDOPA

¹H NMR (500 MHz, D₂O): δ 7.56 (s, 1H), 6.76 (s, 1H), 2.98-4.70 (m, 777H)); ¹³C NMR (500 MHz, D₂O): δ 175.9, 171.8, 150.4, 143.5, 140.6, 127.5, 119.3, 113.2, 67.7-71.9, 58.2, 53.5, 35.2.

### PEG(M_{w} 5 kDa)-nitrodopamine

¹H NMR (500 MHz, D₂O): δ 7.44 (s, 1H), 6.70-6.74 (d, *J*=22.0 Hz, 1H), 3.85-3.91 (m, 6H), 3.47-3.74 (m, 1127H), 3.29 (s, 9H), 3.07 (m, 2H), 3.02 (d, *J*=5.6 Hz, 2H); ¹³C NMR (500 MHz, D₂O): δ 293.9, 177.9, 172.5, 150.6, 119.0, 113.3,69.7-71.1,58.2,39.0,32.5.

### PEG(M_{w} 5 kDa)-mimosine

¹H NMR (500 MHz, D₂O): δ 7.47-7.75 (m, 2H), 6.74-6.75 (d, *J*=6.6 Hz, 1H), 4.24-4.29 (q, *J*=8.6 Hz, 1H), 4.07 (s, 1H), 3.90-3.99 (q, *J*=15.9 Hz, 2H), 3.29-3.74.29 (m, 648H); ¹³C NMR (500 MHz, D₂O): δ 175.1, 172.7, 166.8,, 146.2, 139.0, 128.0, 121.2, 113.9, 68.2-71.1, 59.0, 58.2, 54.1.

### PEG(M_{w} 5 kDa)-DOPA

¹H NMR (500 MHz, D₂O): δ 6.73-6.78 (d, *J*=7.9 Hz, 1H), 6.67 (s, 1H), 6.59-6.60 (d, *J*=7.9 Hz, 1H), 4.42 (m, 1H), 3.96 (s, 2 H), 3.83-3.87 (d, *J*=15.8, 2H), 3.47-3.75 (m, 655 H), 3.29 (s, 5H); ¹³C NMR (500 MHz, D₂O): δ 176.6, 171.8, 144.1, 143.0, 129.7, 121.6, 117.0, 116.2, 69.1-71.1, 58.2, 55.0, 36.5

### PEG(M_{w} 5 kDa)-dopamine

¹H NMR (500 MHz, D₂O): δ 6.74-6.75 (d, *J*=7.3 Hz, 1H), 6.69 (s, 1H), 6.60-6.61 (d, *J*=6.3 Hz, 1H), 3.28-3.97 (m, 517 H), 2.64 (s, 3H); ¹³C NMR (500 MHz, D₂O): δ 172.3, 144.2, 142.7, 131.7, 121.2, 116.7, 116.3,69.0-71.1,58.2,40.4, 33.9

### PEG(M_{w} 5 kDa)-hydroxydopamine

¹H NMR (500 MHz, D₂O): δ 6.33 (s, 1H), 6.29, (s, 1H), 4.15-4.16 (t, *J*=6.6 Hz, 1H) 3.91-3.92 (d, *J*=3.8 Hz, 1H), 3.29-3.68 (m, 362H); ¹³C NMR (500 MHz, D₂O): δ 176.5, 172.6, 145.7, 131.5, 128.9, 108.6, 68.1-71.1, 64.0, 62.3, 53.3, 40.7, 32.4

### PEG(M_{w} 5 kDa)-hydroxypyridine

¹H NMR (500 MHz, D₂O): δ 7.49 (s, 1H), 6.45 (s, 1H), 4.11 (s, 2H), 3.29-3.92 (m, 765H), 2.35 (s, 3H); ¹³C NMR (500 MHz, D₂O): δ 173.2, 173.2, 115.8, 144.9, 142.1, 115.8, 68.2-71.1, 58.2, 52.9, 38.2, 18.4.

### PEG(M_{w} 5 kDa)-hydroxypyrone

¹H NMR (500 MHz, D₂O): δ 4.11 (s, 2H), 3.11-3.96 (m, 478H); ¹³C NMR (500 MHz, D₂O): δ 176.5, 173.2, 165.8, 144.9, 142.1, 115.8,68.2-71.1,58.2, 52.9, 38.2, 18.4.

### PEG(M_{w} 5 kDa)-COOH

¹H NMR (500 MHz, D₂O): δ 4.70 (s, 1H), 6.41 (s, 1H), 4.31 (s, 3H), 4.07 (s, 3H), 4.02 (s, 4H), 3.29-3.74 (m, 977H); ¹³C NMR (500 MHz, D₂O): δ 174.8, 68.1-71.2, 58.2.

While there are shown and described presently preferred embodiments of the invention, it is to be distinctly understood that the invention is not limited thereto but may be otherwise variously embodied and practiced within the scope of the following claims.

### References

[1] Amstad, E. et al., Surface Functionalization of Single Superparamagnetic Iron Oxide Nanoparticles for Targeted Magnetic Resonance Imaging. Small 5 (11), 1334-1342 (2009).
[2] Xu, C.J. et al., Dopamine as a robust anchor to immobilize functional molecules on the iron oxide shell of magnetic nanoparticles. Journal of the American Chemical Society 126 (32), 9938-9939 (2004).
[3] Xie, J., Xu, C., Kohler, N., Hou, Y., & Sun, S., Controlled PEGylation of monodisperse Fe3O4 nanoparticles for reduced non-specific uptake by macrophage cells. Advanced Materials 19 (20), 3163-+ (2007).
[4] Gu, H.W., Yang, Z.M., Gao, J.H., Chang, C.K., & Xu, B., Heterodimers of nanoparticles: Formation at a liquid-liquid interface and particle-specific surface modification by functional molecules. Journal of the American Chemical Society 127 (1), 34-35 (2005).
[5] Gao, J.H. et al., Multifunctional yolk-shell nanoparticles: A potential MRI contrast and anticancer agent. Journal of the American Chemical Society 130 (35), 11828-11833 (2008).
[6] Shultz, M.D., Reveles, J.U., Khanna, S.N., & Carpenter, E.E., Reactive nature of dopamine as a surface functionalization agent in iron oxide nanoparticles. Journal of the American Chemical Society 129 (9), 2482-2487 (2007).
[7] Araujo, P.Z., Morando, P.J., & Blesa, M.A., Interaction of catechol and gallic acid with titanium dioxide in aqueous suspensions. 1. Equilibrium studies. Langmuir 21 (8), 3470-3474 (2005).
[8] Dalsin, J.L. et al., Protein resistance of titanium oxide surfaces modified by biologically inspired mPEG-DOPA. Langmuir 21 (2), 640-646 (2005).
[9] Butterworth, M.D., Illum, L., & Davis, S.S., Preparation of ultrafine silica-and PEG-coated magnetite particles. Colloids and Surfaces a-Physicochemical and Engineering Aspects 179 (1), 93-102 (2001).

## Claims

1. A dispersant comprising at least one anchor group A, and at least one linear or branched spacing group R covalently linked to each other directly or via a connecting group,
said anchor group A being an unsaturated at least partially conjugated substituted six-membered homocycle or heterocycle,
said anchor group A comprising two surface interacting oxygen or hydroxy substituents on adjacent carbon atoms in the ring,
said homocyclic ring comprising at least one further electronegative substituent on the ring, in the case of a further hydroxy substituent in meta position to the spacing group at least two electronegative substituents
said heterocyclic ring comprising one or two, and preferably one, nitrogen and optionally at least one electronegative substituent on the ring,

2. The dispersant of claim 1, wherein the electronegative group is selected from the group consisting of F, Cl, Br, I, NO₂, COOH, OH, SO₄, PO₄.

3. The dispersant of claim 1 or 2, wherein the spacing group R is a group -Sₙ-Fₘ, wherein S is a linear or branched spacer, F is a functional group that is independently from each other selected from the group consisting of functionalities E, terminating groups T, such as methyl groups and hydrogen, anchor groups A and combinations thereof, n and m are integers, n is selected from 0 or 1, m corresponds to the number of branches of S + 1 and is at least 1.

4. The dispersant of any one of the preceding claims, wherein the anchor group is a catechol, quinone and/or semiquinone derivative of formula (I), 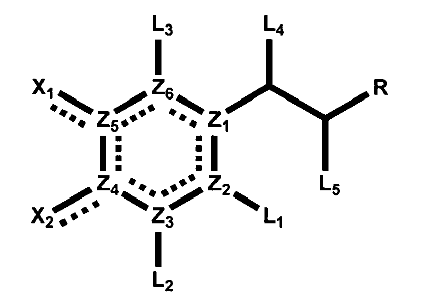 wherein
X₁ and X₂ are independently selected from the group consisting of O and OH,
Z₁-Z₆ are carbons,
the substituents L₁-L₅ are independently from each other selected from hydrogen and electronegative groups, with the provisos that at least one of the substituents L₁-L₃ is an electronegative group, and that in case L₂ = OH, L₁ and/or L₃ are an additional electronegative group, and wherein said anchor group comprises a covalently linked connecting group -C(L4)-C(L5)- for connecting the spacing group R to the anchor group, and
said spacing group R being covalently linked to said anchor group via said connecting group.

5. The dispersant of any one of claims 1 to 3, wherein the anchor group is a heterocycle selected from pyridines derivatives wherein Z₁, Z₂, Z₃ and Z₆ are selected from the group consisting of C and N, Z₄ and Z₅ are C, L₁ - L₃ are selected from the group consisting of hydrogen and electrophilic substituents, and L₄ and L₅ are independently from each other selected from hydrogen and electrophilic substituents, e.g. COOH, wherein preferred heterocycles are mimosine and/or hydroxypyridines.

6. The dispersant of any one of the preceding claims, wherein the spacing group R is covalently linked to the connecting group of the anchor group e.g. through amide, ester, urethane or azide bindings.

7. The dispersant of claim 6, wherein the spacing group (R) is a group Sₙ-Fₘ wherein n and m are as defined above and wherein
the spacer (S) is selected from the group consisting of hydrophilic, biocompatible polymers and/or oligomers including but not exclusively poly(ethylene-glycol), linear or branched or dendritic polymer chains, hydrophobic chain spacers including but not limited to alkyl chains, responsive polymers e.g. thermo-, light- or pH-responsive polymers including but not limited to e.g. poly(N-isopropylacrylamide), and poly(methyl oxazolines), amino acids, peptides and oligopeptides, and amphiphilic block-copolymers and/or wherein
the functionality (E) is selected from ligands for targeting purposes such as biotin, antibodies, proteins, DNA, RNA, aptamers and/or Fab fragments, ligands for additional imaging modalities such as, but not limited to, fluorophores, quantum dots, radioactive tracers, MR active ions, Raman and/or IR active groups, ligands for further binding and crosslinking including but not limited to acrylates and ion complexers like terpyridin, and at least one further anchor group as defined above.

8. The dispersant of any one of the preceding claims, comprising more than one anchor group per molecule.

9. The dispersant of any one of the preceding claims wherein the anchor groups and/or the spacing groups are independently from each other selected from anyone of the preceding claims.

10. The dispersant of any one of claims 1 to 8 which consists of one kind of anchor group and one kind of spacing group.

11. A dispersant composition comprising several dispersants of any one of the preceding claims, i.e. varying anchor groups and/or varying spacing groups.

12. A method for individually stabilizing and/or functionalizing inorganic non-metallic particles, wherein said particles are stabilized and/or functionalized with a dispersants of anyone of claims 1 to 12 by using
grafting from techniques, such as free radical polymerization from initiators irreversibly immobilized on the particle surface group using an anchor group as defined in any one of the preceding claims,
grafting to techniques, such as co-adsorption of dispersants of one or different kinds and/or of dispersants which have the same anchor group but different functionalization to generate particles with different functionalizations,
co-adsorption of dispersants in specific molar ratios of differently functionalized and/or functionalized and unfunctionalized dispersants in ratios suitable for tuning the surface density, wherein said functionalized dispersants may already be functionalized with the desired functional group and/or contain a reactive end group and then linking the desired functionality to the reactive end of dispersants of already stabilized particles.

13. A method for tailoring the particle size, stability, zeta potential and the inter-particle interactions by individually controlling the core size and choosing the appropriate molecular weight and/or molecular configuration (e.g. linear or dendritic) of the spacer molecules in the dispersant and/or salt concentration and/or solvent quality with respect to the spacer molecule.

14. Inorganic non-metallic particles with an inorganic non-metallic core, such as oxides, hydroxide-oxides, carbides and nitrides, in particular an inorganic core selected from the group consisting of FeOₓ, FeOOH, FeCₓ and mixtures thereof, comprising at least one dispersant of any one of claims 1 to 11 adsorbed on their surface, said particles optionally having dynamic shell structure.

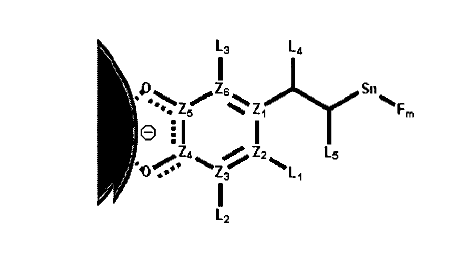

15. Use of inorganic non-metallic particles of claim 14
in hyperthermia e.g. for but not limited to cancer treatment using alternating magnetic fields on magnetic particles, or
for generating heat by applying an AC magnetic field on magnetic particles suitable for the creation of smart and/or reversibly responsive materials, or
for generating heat by application to high intensity electromagnetic fields, e.g. by laser irradiation
for the creation of nano, meso and microporous materials by any deposition method using solvents requiring stably dispersed particles using hydrophobic spacers, including but not limited to alkanes, for non-polar solvents and, hydrophilic polymer brushes, including but not limited to poly(ethylene glycol) for polar solvents,
for cross-linking inorganic non-metallic particles, of the same or different materials, using a dispersant having at least two anchor groups which can be the same or different, and which are covalently linked via a spacer, e.g. via a spacer that has multiple branching points suitable for covalently binding multiple and/or different anchor groups.

16. Use of inorganic non-metallic particles stabilized with dispersants according to any one of claims 1 to 11, for forming highly concentrated particle suspensions which are important for processing of inorganic non-metallic materials, in particular for use as ferrofluids, and ferroelectric fluids and materials and/or for being used for data storage purposes.
